(19) Europäisches Patentamt
European Patent Office
Office européen des brevets

(11) Publication number: **0 031 654**
**B1**

(12) **EUROPEAN PATENT SPECIFICATION**

(45) Date of publication of patent specification: **16.02.83**

(51) Int. Cl.³: **C 07 C 2/84,** C 07 C 15/52

(21) Application number: **80304414.8**

(22) Date of filing: **08.12.80**

(54) **Dehydrocoupling of toluene.**

(30) Priority: **10.12.79 US 101941**
**10.12.79 US 101943**
**10.12.79 US 101945**
**10.12.79 US 101982**
**10.12.79 US 101928**
**10.12.79 US 101921**
**10.12.79 US 101944**

(43) Date of publication of application:
**08.07.81 Bulletin 81/27**

(45) Publication of the grant of the patent:
**16.02.83 Bulletin 83/7**

(84) Designated Contracting States:
**DE FR GB IT**

(56) References cited:
**GB - A - 1 259 763**
**GB - A - 1 259 766**
**US - A - 3 476 747**
**US - A - 3 557 235**
**US - A - 3 965 206**
**US - A - 4 091 044**

**CHEMICAL ABSTRACTS, vol. 84, no. 13, 29—03—1976, page 477, nr. 89581v Columbus, Ohio, US**

(73) Proprietor: **MONSANTO COMPANY**
**Patent Department 800 North Lindbergh Boulevard**
**St. Louis, Missouri 63166 (US)**

(72) Inventor: **Williamson, Alex Nathan**
**300 Leland Drive**
**Greensboro North Carolina 27408 (US)**
Inventor: **Tremont, Samuel Joseph**
**522 Springmeadow Drive**
**Manchester Missouri 63011 (US)**
Inventor: **Solodar, Arthur John**
**8135 Cornell Court**
**University City Missouri 63130 (US)**

(74) Representative: **McLean, Peter et al,**
**Monsanto Europe S.A. Patent Department Avenue de Tervuren 270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 031 654 B1**

Courier Press, Leamington Spa, England

Dehydrocoupling of toluene background of the invention

Field of the Invention

This invention relates to the production of dehydrocoupled toluene products. It is particularly related to the oxidative synthesis of 1,2-diphenylethylene (stilbene) from toluene and to inorganic metal/oxygen compositions effective for oxidatively coupling toluene to produce stilbene.

Stilbene, because of its unsaturated character is very reactive and may be employed in various organic sytheses. Derivatives of stilbene are useful in the production of products which may be used in the manufacture of dyes, paints and resins. It is also useful in optical brighteners, in pharmaceuticals, and as an organic intermediate.

Description of the Prior Art

Dehydrocoupling of toluene by the reaction with lead oxide to form stilbene has been reported by Behr and Van Dorp, *Chem. Ber., 6,* 753 (1873) and Lorenz, *Chem. Ber., 7,* 1996 (1874). In this reported work, stilbene is obtained by conveying toluene over lead oxide maintained at or about a dark red glow. The coupling of toluene using elemental sulfur as the coupling agent has been reported by Renard, *Bull. Soc. Chim. France, 3,* 958 (1889); *5,* 278 (1891) and the types of products produced by such coupling reactions have been discussed by Horton, *J. Org. Chem., 14,* 761 (1949). More recently, U.S. Patent No. 3,476,747 discloses arsenic pentoxide, antimony tetroxide, antimony tetroxide, antimony pentoxide, bismuth trioxide, and manganese arsenate as oxidants for the oxidative dehydrocoupling of toluene to form 1,2-bis(aryl)ethylenes. Similarly, U.S. Patent No. 3,494,956 discloses lead oxide, cadmium oxide, and thallium oxide as suitable oxidants, and in Example 9 a mixture of toluene and oxygen passed over heated lead oxide produced bibenzyl. In U.S. Patent No. 3,557,235 the stoichiometric toluene coupling reaction is taught using an oxide of bismuth, lead, tellurium, barium, thallium, cadmium, or mixtures thereof which serves as the source of oxygen in the reaction. U.S. Patent No. 3,963,793 teaches the use of bismuth trioxide and thallium trioxide or mixtures thereof supported on basic carrier materials selected from the oxides of Group 2a elements and having a minimum surface area of 20 $mg^2/g$ as suitable for toluene coupling to produce bibenzyl. The addition to the supported catalyst of small amounts of silver as a promoter is also disclosed. In U.S. Patent No. 3,965,206 oxides of lead, cadmium, bismuth, and mixtures thereof are taught as suitable oxidants for toluene coupling. This patent also teaches the disproportionation of the stilbene with ethylene to produce styrene. U.S. Patent No. 3,980,580 discloses an oxygen composition of lead, magnesium, and aluminium as an oxidant for toluene coupling. Also, U.S. Patent No. 4,091,044 discloses oxygen compositions of lead and antimony and optionally with bismuth as oxidants for toluene coupling to form stilbene.

## SUMMARY OF THE INVENTION

This invention is directed to a process for the oxidative dehydrocoupling of toluene and toluene derivatives to stilbene and stilbene derivatives. In another aspect, this invention is directed to inorganic metal/oxygen compositions which are useful as the oxygen source for the oxidative dehydrocoupling of toluene to produce stilbene, or alternatively as catalysts or combination catalysts/oxygen source for the dehydrocoupling reaction when oxygen or an oxygen-containing gas is heated with the toluene.

Accordingly, typical objects of this invention are to provide (1) inorganic metal/oxygen compositions useful as the oxygen source in oxidative dehydrocoupling of toluene and toluene derivatives, (2) inorganic metal/oxygen compositions useful as catalysts in the oxidative dehydrocoupling of toluene and toluene derivatives, (3) inorganic metal/oxygen compositions useful as combination catalysts/oxygen source in the oxidative dehydrocoupling of toluene and toluene derivatives, (4) a one-step, vapor phase process for the production of stilbene and stilbene derivatives and bibenzyl and bibenzyl derivatives, and (5) a one-step, vapor phase dehydrocoupling process for converting toluene and toluene derivatives to stilbene and stilbene derivatives characterized by high toluene conversions and high stilbene selectivities.

These and other objects and advantages of this invention are achieved by the process disclosed herein for dehydrocoupling toluene and toluene derivatives. Toluene dehydrocoupled products are produced by heating toluene (and toluene derivatives) in the presence of an inorganic metal/oxygen composition which functions in a catalytic mode, a stoichiometric mode as an oxidant or oxygen carrier, or a combined catalytic/stoichiometric mode for the dehydrocoupling reaction.

## DESCRIPTION OF THE PREFERRED EMBODIMENTS

In accordance with this invention, toluene and toluene derivatives are dehydrocoupled by a process which comprises contacting the toluene (and toluene derivatives) in the vapor phase at a temperature between 450°C. and 650°C. with an inorganic metal/oxygen composition characterized by an empirical formula selected from:

(a) $$Pb\, M^1_a\, M^2_b\, O_x$$

where $M^1$ is at least one element selected from silver, zinc, gallium, indium, thallium, germanium, phosphorus, arsenic, thorium, the lanthanides, Groups 1a, 2a, 3b, 4b and 8 of the Periodic Table of the Elements and mixtures thereof, and $M^2$ is an element selected from bismuth and antimony, and wherein a is 0.01 to 10, b is 0 to 10, and x is a number taken to satisfy the average valences of lead, $M^1$ and $M^2$ in the oxidation states in which they exist in the composition, with the proviso that when $M^2$ is bismuth, $M^1$ cannot be gallium or thallium; and when $M^2$ is antimony, $M^1$ cannot be indium, thallium, germanium, phosphorus, or thorium; and

(b) $$Bi\ M^3_a\ M^4_b\ O_x$$

where $M^3$ is at least one element selected from zinc, germanium, thorium, the lanthanides, Groups 1a, 3b, 4b, and 8 of the Periodic Table of the Elements and mixtures thereof, and $M^4$ is at least one element selected from indium, silver, Group 2a of the Periodic Table of the Elements, and mixtures thereof, and wherein a is 0.01 to 10, b is 0 to 10, and x is a number taken to satisfy the average valencies of bismuth, $M^3$, and $M^4$ in the oxidation state in which they exist in the composition to yield the toluene dihydrocoupled toluene product.

The inorganic metal/oxygen composition functions in a catalytic mode, a stoichiometric mode as an oxidant or oxygen carrier, or a combined catalytic/stoichiometric mode for the dehydrocoupling of toluene.

In the catalytic mode of operation, oxygen or an oxygen-containing gas such as air or oxygenenriched air is reacted with toluene in the presence of the inorganic metal/oxygen composition in an amount sufficient for the dehydrocoupling reaction. In the stoichiometric mode of operation, the inorganic metal/oxygen composition is the sole source of oxygen. That is, in the latter instance the dehydrocoupling of toluene is conducted in the substantial absence of added free oxygen such as would be obtained from air. In the combined catalytic/stoichiometric mode of operation, oxygen or an oxygen-containing gas is added as a reactant in a manner similar to that noted hereinabove for the catalytic mode of operation. However, the amount of added oxygen is not sufficient for the dehydrocoupling reaction and the required additional oxygen must be supplied by the inorganic metal/oxygen composition.

Of these three modes of operation, the stoichiometric mode is generally preferred in that undesirable side reactions — oxidative dealkylation, for example, to produce benzene and carbon dioxide — are substantially reduced. It will, of course, be recognized that in spite of the undesirability of producing benzene during the course of the reaction of the present process, benzene is a valuable article of commerce. It is therefore highly desirable to recover the benzene values when substantial production thereof occurs. The recovery and purification of such benzene values may be accomplished by any standard method and means known to the art.

The term "dehydrocoupling" and related terms are employed herein to mean that the toluene molecules are coupled or dimerized — with carbon-carbon bond formation occurring between the methyl group carbons — and the coupled molecules have lost either one or two hydrogen atoms from the methyl group of each toluene molecule. When two hydrogen atoms per molecule of toluene are lost, the carbon-carbon bond at the coupling or dimerization site is unsaturated as by dehydrogenation, that is, stilbene is the product. On the other hand, bibenzyl, having a saturated carbon-carbon bond at the coupling site, is the product when only one hydrogen atom per molecule of toluene is lost.

In general, the production of stilbene as the dehydrocoupled toluene product is preferred over the production of bibenzyl. This stated preference is due to the unsaturated character of stilbene as opposed to the saturated character of bibenzyl. And, as is well known in the art, the presence of the unsaturated olefinic carbon-carbon double bond causes the stilbene to exhibit high reactivity, thereby facilitating its direct use as an organic intermediate in numerous organic syntheses.

The process of this invention is conveniently carried out in an apparatus of the type suitable for carrying out chemical reactions in the vapor phase. It can be conducted in a single reactor or in multiple reactors using either a fixed bed, a moving bed, or a fluidized bed system to effect contacting of the reactant or reactants and inorganic metal/oxygen composition. The reactant toluene or toluene derivative will generally be heated and introduced into the reactor as a vapor. However, the reactant may be introduced to the reactor as a liquid and then vaporized.

The oxidative dehydrocoupling reaction is carried out in the vapor phase and under the influence of heat. The temperature range under which the reaction can be carried out ranges from about 450°C. to about 650°C. and preferably is conducted at from about 500°C. to about 600°C.

Pressure is not critical in the process of this invention. The reaction may be carried out at subatmospheric, atmospheric, or superatmospheric pressures as desired. It will be generally preferred, however, to conduct the reaction at or near atmospheric pressure. Generally, pressures from about $2.53 \times 10^4$ pascals or Pa (0.25 atmosphere or atm) to about $4.05 \times 10^5$ Pa (4.0 atm) may be conveniently employed.

The reaction time for the contact of the reactant with the inorganic metal/oxygen composition in this invention may be selected from a broad operable range which may vary from about 0.1 to about 60 seconds. The reaction time may be defined as the length of time in seconds which the reactant gases

measured under reaction conditions are in contact with the inorganic metal/oxygen composition in the reactor. The reaction time may vary depending upon the reaction temperature and the desired toluene conversion level. At higher temperatures and lower toluene conversion levels, shorter contact times are required. Generally, the contact time will vary from about 0.5 second to about 20 seconds. Preferable, for optimum conversion and selectivity in the preferred temperature range, a contact time from about 1 second to about 12 seconds is employed.

In addition to the toluene and/or toluene derivatives, other inert substances such as nitrogen, helium, and the like may be present in the reaction. Such inert materials may be introduced to the process alone or may be combined with the other materials as feed. Water or steam may be added to the reaction zone, preferably being introduced with the feed in order to improve the selectivity to the desired products and particularly to suppress complete oxidation to $CO_2$. Steam-to-hydrocarbon ratios in the range from 0.1 to 10 or more are suitable, the upper limit being determined by practical cost considerations. Ratios in the range from 0.5 to 3 are preferred.

The inorganic metal/oxygen composition suitable for use in this invention contains oxygen in such a manner that it is capable of releasing stoichiometric quantities of oxygen under the oxidative reaction conditions employed. The oxygen in the composition is associated with the metals present, or as mixtures of oxides and complexes. The inorganic metal/oxygen composition can be characterized by an empirical formula selected from:

(a)

$$Pb\ M^1_a\ M^2_b\ O_x$$

where $M^1$ is at least one element selected from silver, zinc, gallium, indium, thallium, germanium, phosphorus, arsenic, thorium, the lanthanides, Groups 1a, 2a, 3b, 4b and 8 of the Periodic Table of the Elements and mixtures thereof, and $M^2$ is an element selected from bismuth and antimony, and wherein a is 0.01 to 10, b is 0 to 10, and x is a number taken to satisfy the average valences of lead, $M^1$ and $M^2$ in the oxidation states in which they exist in the composition, with the proviso that when $M^2$ is bismuth, $M^1$ cannot be gallium or thallium; and when $M^2$ is antimony, $M^1$ cannot be indium, thallium, germanium, phosphorus, or thorium; and

(b)

$$Bi\ M^3_a\ M^4_b\ O_x$$

where $M^3$ is at least one element selected from zinc, germanium, thorium, the lanthanides, Groups 1a, 3b, 4b and 8 of the Periodic Table of the Elements and mixtures thereof, and $M^4$ is at least one element selected from indium, silver, Group 2a of the Periodic Table of the Elements, and mixtures thereof, and wherein a is 0.01 to 10, b is 0 to 10, and x is a number taken to satisfy the average valencies of bismuth, $M^3$, and $M^4$ in the oxidation state in which they exist in the composition to yield the toluene dehydrocoupled toluene product. Preferred compositions for formula (a) and formula (b) are those wherein a is 0.5 to 5, b is 0.5 to 5 and x is a number taken to satisfy the average valences of $M^1$ and $M^2$ in the oxidation states in which they exist in the composition.

Of the $M^1$ elements listed for formula (a), silver, zinc, lithium, sodium, potassium, rubidium, cesium, magnesium, calcium, strontium, barium, indium, arsenic, the lanthanides and those from Groups 3b, 4b and 8 of the Periodic Table of the Elements, and mixtures thereof, are preferred, with silver, zinc, potassium, zirconium, barium, calcium, strontium, nickel, platinum and cerium, and mixtures thereof, being most preferred.

Of the $M^3$ elements listed for formula (b), zinc, the lanthamides and those from Groups 3b, 4b and 8 of the Periodic Table of the Elements, and mixtures thereof are preferred, with zinc, cerium, zirconium, cobalt, nickel and platinum being most preferred. For $M^4$ of formula (b) indium, silver, magnesium, calcium, strontium and barium, and mixtures thereof, are preferred, with silver, calcium, strontium and barium being most preferred.

The term "Periodic Table of the Elements" as employed herein refers to the Periodic Table of the Elements published in *CRC Handbook of Chemistry and Physics,* 59th ed., Weast, Ed., CRC Press, Inc., West Palm Beam, FL, 1978, Inside Front Cover.

The inorganic metal/oxygen composition may be employed in this invention alone or in association with a support or carrier. The use of a support may be particularly advantageous where the composition is too soft or attrition-prone to retain its structural integrity during reactor charging and/or under reaction conditions encountered during the course of the reaction process. Suitable supports for the compositions are, for example, silica, alumina, silica-alumina, metal aluminates such as magnesium aluminate, calcium aluminate, and the like.

As noted hereinafter, the dehydrocoupling reaction may be conducted in the presence or absence of added free oxygen. When oxygen is not added to the system, that is, the reaction is conducted in the stoichiometric mode of operation, the oxygen required for the reaction is provided by the inorganic metal/oxygen composition which enters into the reaction and is consequently reduced (or, in actual practice, partially reduced) during the course of the reaction. This necessitates regeneration or re-oxidation which can be easily effected by heating the material in air or oxygen at temperatures from about 500°C. to about 650°C. for a period of time ranging from about 5 seconds to about one hour. In

a semi-continuous operation, regeneration can be effected by periodic interruption of the reaction for re-oxidation of the reduced composition, that is, periods of reaction are cycled with periods of regeneration. Operation, however, can be on a continuous basis whereby a portion of the inorganic metal/oxygen composition can be continuously or intermittently removed, re-oxidized and the re-oxidized material can thereafter be continuously or intermittently returned to the reaction. The latter method is particularly adapted to operations in which the inorganic metal/oxygen composition is fed in the form of a fluidized bed or a moving bed system.

When oxygen is employed as a reactant, the reaction may be conducted in either a catalytic mode of operation or a combined catalytic/stoichiometric mode of operation, depending on the amount of oxygen supplied. In the catalytic mode of operation, oxygen is supplied in an amount sufficient for the dehydrocoupling reaction. The actual amount of oxygen supplied may be specified as a function of the amount of the toluene or other suitable hydrocarbon component. On this basis the amount of oxygen supplied is ordinarily selected to provide a hydrocarbon-to-oxygen mole ratio from about 1 to about 8 and preferably from about 2 to about 6.

In the combined catalytic/stoichiometric mode of operation, the amount of oxygen supplied as a reactant is not sufficient for the dehydrocoupling reaction, thereby requiring an additional source of oxygen. The required additional oxygen will be supplied by the inorganic metal/oxygen composition, that is, the composition will serve as the additional source of oxygen. As a result, the inorganic metal/oxygen composition enters into the reaction and is consequently reduced during the course of the reaction. This necessitates regeneration or re-oxidation of the reduced composition which can be easily effected as described hereinabove for the stoichiometric mode of operation.

In either mode of operation employing added oxygen as a reactant, whether catalytic or combined catalytic/stoichiometric, the added free oxygen may be supplied either as oxygen or an oxygen-containing gas such as air or oxygen/enriched air.

The inorganic metal/oxygen compositions can be prepared in several ways. The simplest method involves intimately mixing the powdered metal oxides in the dry state and calcining. Another method involves adding the metal oxides to water with stirring, filtering to remove excess water or, alternatively, heating to evaporate the water, drying, and calcining. In another method of preparation, the powdered metal oxides can be intimately mixed before forming a paste of them with water and further mixing the paste. The paste can be spread and dried in air, after which it can be calcined in air. The calcined product can then be crushed and sieved to the desired mesh size. In still another method of preparation, the powdered metal oxides can be mixed in the dry state together with a material which facilitates forming the mixture into pellets and then pressed to form pellets which are calcined prior to use. A further method of preparation involves intimately mixing the powdered metal oxides in water and spray drying the resulting slurry or solution to produce relatively dust-free and free-flowing spherical particles which are also calcined prior to use.

In an alternative method of preparation, suitable inorganic metal/oxygen composition precursor salts such as nitrates, carbonates, and acetates are intimately mixed or dissolved in water or nitric acid and heated to thermally decompose the precursor salts to form the corresponding oxides and/or oxygen complexes. The oxides and/or oxygen complexes can then be treated as described hereinabove prior to use.

Temperatures employed for calcination of the inorganic metal/oxygen composition may vary from about 400°C. to about 1200°C. The higher temperatures from about 900°C. to about 1100°C. result in higher selectivity with some loss in activity. Preferred calcination temperatures, therefore, lie in the range from about 700°C. to about 1000°C. Calcination times may vary from about 1 hour to about 12 hours or more and preferably from about 2 hours to about 10 hours at the higher temperatures. The surface area of the composition is not critical. In general, however, a surface area less than about 10 $m^2/g$ is preferred, with values between about 0.1 $m^2/g$ and about 5 $m^2/g$ being most preferred.

As previously indicated, the process of this invention is preferably carried out in the absence of added free oxygen, that is, in the stoichiometric mode of operation, and utilizes only that oxygen supplied by the inorganic metal/oxygen composition. Also, with few exceptions, at substantially comparable conditions, the lower the toluene conversion level, the higher will be the selectivity to the dehydrocoupled products. That is, under similar conditions, the selectivity to the dehydrocoupled toluene products is in general inversely proportional to the toluene conversion level. However, for practical reasons, the dehydrocoupling reaction will generally be conducted at a toluene conversion level of about 20 to about 55 percent.

The dehydrocoupled toluene products, stilbene and bibenzyl, may be recovered and purified by any appropriate method and means known to the art and further elucidation here will be unnecessary duplication of the art. As noted previously, stilbene, of course is the preferred product.

The following specific examples illustrating the best presently-known methods of practicing this invention are described in detail in order to facilitate a clear understanding of the invention. It should be understood, however, that the detailed expositions of the application of the invention, while indicating preferred embodiments, are given by way of illustration only and are not to be construed as limiting the invention.

Example 1

*Procedure A* — A series of inorganic metal/oxygen compositions having varied Pb/$M^1$ atomic ratios were prepared by intimately mixing the appropriate amount in grams of lead (II) oxide (PbO) and at least one $M^1$ oxide (or hydroxide) in water, filtering to remove excess water or, alternatively, heating to evaporate the water. The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 6 below. The parameters for such compositions, conveniently designated as 1—A—, are set forth in Table 1.

TABLE 1

| SAMPLE NO. | $PbM^1_aO_x$ | STARTING MATERIALS GRAMS (MOLES) Pb | M¹ | ATOMIC RATIO Pb/M¹ | CALCINATION CONDITIONS TEMPERATURE, °C./TIME, HOURS INITIAL | FINAL |
|---|---|---|---|---|---|---|
| 1—A—1— | $PbZr_aO_x$ | PbO | $ZrO_2$ | Pb/Zr | | |
| (a) | $PbZr_{0.33}O_x$ | 90.0 (0.40) | 16.6 (0.13) | 3 | 400/2 | 700/12 |
| (b) | $PbZr_{0.5}O_x$ | 80.0 (0.36) | 22.1 (0.18) | 2 | 400/2 | 700/12 |
| (c) | $PbZrO_x$ | 70.0 (0.31) | 38.7 (0.31) | 1 | 400/2 | 700/12 |
| (d) | $PbZr_2O_x$ | 50.0 (0.22) | 55.2 (0.45) | 0.5 | 400/2 | 700/12 |
| (e) | $PbZr_3O_x$ | 40.0 (0.18) | 66.3 (0.54) | 0.33 | 400/2 | 700/12 |
| 1—A—2— | $PbK_aO_x$ | PbO | 85% KOH | Pb/K | | |
| (a) | $PbK_2O_x$ | 55.0 (0.25) | 33.0 (0.50) | 0.5 | 350/2 | 700/6 |
| 1—B—1— | $PbY_aO_x$ | PbO | $Y_2O_3$ | Pb/Y | | |
| (a) | $PbY_{0.33}O_x$ | 38.0 (0.17) | 6.4 (0.028) | 3 | 400/12 | 700/12 |
| (b) | $PbY_{0.5}O_x$ | 36.0 (0.16) | 9.0 (0.040) | 2 | 400/12 | 700/12 |
| (c) | $PbYO_x$ | 30.0 (0.13) | 15.0 (0.066) | 1 | 400/12 | 700/12 |
| (d) | $PbY_6O_x$ | 11.2 (0.050) | 33.9 (0.15) | 0.17 | 400/12 | 700/12 |
| (e) | $PbY_{10}O_x$ | 9.0 (0.040) | 45.4 (0.20) | 0.1 | 400/12 | 700/12 |
| 1—B—2— | $PbHo_aO_x$ | PbO | $Ho_2O_3$ | Pb/Ho | | |
| (a) | $PbHoO_x$ | 11.8 (0.053) | 10.0 (0.026) | 1 | 450/1 | 800/8 |

*Procedure B* — The procedure described in Procedure A above was employed except that following the final calcination, the calcined material was removed from the oven, cooled, crushed, and mixed with a calcium aluminate cement (Alcoa—CA—25) in an amount corresponding to 25% by weight of the dry weight of the calcined material. The resulting solid mixture was slurried with water to form a thick paste and allowed to air dry. The air-dried paste was calcined in air in an open casserole dish for 2 hours at 400°C. and then at a final temperature of 700°C. for an additional 2 hours. The supported inorganic metal/oxygen composition was removed from the oven, cooled, crushed in a mortar, and sieved to 14/30 mesh particles for evaluation in the toluene conversion reactor described in Example 6 below. The parameters for such compositions, conveniently designated as 1—B—, are set forth in Table 1.

Example 2

*Procedure A* — A series of inorganic metal/oxygen compositions having varied $Pb/M^1$ atomic ratios were prepared by dissolving the appropriate amount in grams of lead (II) acetate trihydrate $[Pb(OCOCH_3)_2.3H_2O]$, lead (II) nitrate $[Pb(NO_3)_2]$, or lead (II) oxide (PbO) and at least one $M^1$ nitrate in approximately 100 milliliters of concentrated nitric acid ($HNO_3$) and heating to evaporate and decompose the nitric acid and the salts. The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 6 below. The parameters for such compositions, conveniently designated as 2—A—, are set forth in Table 2.

*Procedure B* — The procedure described in Procedure A above was employed except that following the final calcination, the calcined material was supported on a calcium aluminate cement (Alcoa—CA—25) as described in Example 1, Procedure B hereinabove. The parameters for such compositions, conveniently designated as 2—B—, are set forth in Table 2.

TABLE 2

| SAMPLE NO. | $PbM^1_aO_x$ | STARTING MATERIALS GRAMS (MOLES) Pb | M¹ | ATOMIC RATIO Pb/M¹ | CALCINATION CONDITIONS TEMPERATURE, °C./TIME, HOURS INITIAL | FINAL |
|---|---|---|---|---|---|---|
| 2—A—1— | $PbCo_aO_x$ | PbO | $Co(NO_3)_2.6H_2O$ | Pb/Co | | |
| (a) | $PbCoO_x$ | 44.6 (0.20) | 59.0 (0.20) | 1 | 450/1 | 750/8 |
| 2—A—2— | $PbTh_aO_x$ | $Pb(OCOCH_3)_2.3H_2)$ | $Th(NO_3)_4.4H_2O$ | Pb/Th | | |
| (a) | $PbThO_x$ | 57.0 (0.15) | 82.8 (0.15) | 1 | 450/1 | 750/8 |
| 2—B—1— | $PbZn_aO_x$ | $Pb(NO_3)_2$ | ZnO | Pb/Zn | | |
| (a) | $PbZn_{0.2}O_x$ | 143.0 (0.43) | 7.0 (0.086) | 5 | 450/1 | 800/4 |
| (b) | $PbZn_{0.33}O_x$ | 135.0 (0.41) | 11.0 (0.14) | 3 | 450/1 | 800/4 |
| (c) | $PbZn_{0.5}O_x$ | 130.0 (0.39) | 16.0 (0.20) | 2 | 450/1 | 800/4 |
| (d) | $PbZnO_x$ | 120.0 (0.36) | 29.4 (0.36) | 1 | 450/1 | 800/4 |
| (e) | $PbZn_2O_x$ | 100.0 (0.30) | 49.0 (0.60) | 0.5 | 450/1 | 800/4 |
| (f) | $PbZn_3O_x$ | 90.0 (0.27) | 66.0 (0.81) | 0.33 | 450/1 | 800/4 |
| (g) | $PbZn_5O_x$ | 70.0 (0.21) | 86.0 (1.057) | 0.2 | 450/1 | 800/4 |

Example 3

*Procedure A* — A solution containing an appropriate amount in grams of lead (II) carbonate ($PbCO_3$) dissolved in 300 milliliters of water and acidified to pH 6 with concentrated nitric acid was mixed with a solution containing an appropriate amount in grams of sodium hydrogen phosphate heptahydrate ($NaHPO_4.7H_2O$; $M^1$ = phosphorus, P) dissolved in 300 milliliters of water. The solutions were stirred at high speeds for 2 hours. The precipitate was collected by suction filtration, dried, and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 6 below. The parameters for such compositions, conveniently designated as 3—A—, are set forth in Table 3.

*Procedure B* — The procedure described in Procedure A above was employed except that sodium hydrogen arsenate heptahydrate ($NaHAsO_4.7H_2O$; $M^1$ = arsenic, As) was employed and following the final calcination, the calcined material was supported on a calcium aluminate cement (Alcoa—CA—25) as described in Example 1, Procedure B hereinabove. The parameters for such compositions, conveniently designated as 3—B—, are set forth in Table 3.

TABLE 3

| SAMPLE NO. | $PbM^1_aO_x$ | STARTING MATERIALS GRAMS (MOLES) | | ATOMIC RATIO $Pb/M^1$ | CALCINATION CONDITIONS TEMPERATURE, °C./TIME, HOURS | |
|---|---|---|---|---|---|---|
| | | Pb | $M^1$ | | INITIAL | FINAL |
| 3—A—1— | $PbP_aO_x$ | $PbCO_3$ | $Na_2HPO_4.7H_2O$ | Pb/P | | |
| (a) | $PbPO_x$ | 129.0 (0.48) | 129.0 (0.48) | 1 | 400/2 | 700/3 |
| 3—B—1— | $PbAs_aO_x$ | $PbCO_3$ | $Na_2HAsO_4.7H_2O$ | Pb/As | | |
| (a) | $PbAsO_x$ | 129.0 (0.48) | 150.0 (0.48) | 1 | 400/2 | 700/12 |

Example 4

*Procedure A* — A series of inorganic metal/oxygen compositions having varied Pb/$M^1$ atomic ratios can be prepared by the following described procedure. Dissolve the appropriate amount in grams of lead (II) nitrate [$Pb(NO_3)_2$] and at least one (water soluble) $M^1$ nitrate in water and heat to evaporate the water and decompose the nitrates. Place the resulting solid in an open casserole dish and calcine in air for an initial period at an initial temperature and then at a final temperature for an additional period (usually 1 hour at 450°C. and 800°C. for 10 hours, respectively). Remove the calcined material from the oven, cool, crush in a mortar, and sieve to 14/30 mesh particles for evaluation in the toluene conversion reactor described in Example 6 below. Following these steps should result in compositions, conveniently designated as 4—A—, having properties beneficial for use in the process of this invention.

*Procedure B* — The procedure described in Procedure A above was employed except that following the final calcination, the calcined material was supported on a calcium aluminate cement (Alcoa—CA—25) as described in Example 1, Procedure B hereinabove. The parameters for such compositions, conveniently designated as 4—B—, are set forth in Table 4.

TABLE 4

| SAMPLE NO. | $PbM^1_aO_x$ | STARTING MATERIALS GRAMS (MOLES) | | ATOMIC RATIO $Pb/M^1$ | CALCINATION CONDITIONS TEMPERATURE, °C./TIME, HOURS | |
|---|---|---|---|---|---|---|
| | | Pb | $M^1$ | | INITIAL | FINAL |
| 4—B—1— | $PbAg_aO_x$ | $Pb(NO_3)_2$ | $AgNO_3$ | Pb/Ag | | |
| (a) | $PbAg_{0.1}O_x$ | 140.0 (0.42) | 7.2 (0.042) | 10 | 450/1 | 800/10 |
| (b) | $PbAg_{0.125}O_x$ | 140.0 (0.42) | 9.0 (0.053) | 8 | 450/1 | 800/10 |
| (c) | $PbAg_{0.17}O_x$ | 80.0 (0.24) | 6.8 (0.040) | 6 | 450/1 | 800/10 |
| (d) | $PbAg_{0.25}O_x$ | 133.0 (0.40) | 17.0 (0.10) | 4 | 450/1 | 800/10 |
| (e) | $PbAg_{0.5}O_x$ | 121.0 (0.37) | 31.0 (0.18) | 2 | 450/1 | 800/10 |

Example 5

*Procedure A* — A series of inorganic metal/oxygen compositions containing two or more $M^1$ elements were prepared by intimately mixing the appropriate amount of lead (II) oxide (PbO), lead (II) carbonate ($PbCO_3$), or lead (II) nitrate [$Pb(NO_3)_2$] with at least two $M^1$ carbonates, nitrates, or oxides (or, alternatively, mixing an appropriate amount of a suitable lead-containing binary metal/oxygen composition with at least one $M^1$ carbonate, nitrate, or oxide) in water and heating to evaporate the water and/or decompose the carbonates and/or nitrates. The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 6 below. The parameters for such compositions, conveniently designated as 5—A—, are set forth in Table 5.

*Procedure B* — The procedure described in Procedure A above was employed except that following the final calcination, the calcined material was supported in a calcium aluminate cement (Alcoa—Ca—25) as described in Example 1, Procedure B hereinabove. The parameters for such compositions, conveniently designated as 5—B—, are set forth in Table 5.

TABLE 5

| SAMPLE NO. | $PbM^1_aO_x$ | STARTING MATERIALS GRAMS (MOLES) | | | CALCINATION CONDITIONS TEMPERATURE, °C./TIME HOURS | |
|---|---|---|---|---|---|---|
| | | Pb | M¹ | | INITIAL | FINAL |
| 5—A—1—(a) | $PbAg_{0.17}Zr_{0.33}O_x$ | PbO | $AgNO_3$ | $ZrO_2$ | — | 700/12 |
| | | 107.1 (0.48) | 13.6 (0.080) | 19.7 (0.16) | | |
| 5—A—2—(a) | $PbK_{0.67}Zr_{0.33}O_x$ | PbO | $K_2CO_3$ | $ZrO_2$ | 400/2 | 700/12 |
| | | 93.7 (0.42) | 19.3 (0.14) | 17.2 (0.14) | | |
| 5—A—3—(a) | $PbY_{0.33}Zr_{0.35}O_x$ | PbO | $Y_2O_3$ | $ZrO_2$ | 400/2 | 700/12 |
| | | 90.0 (0.40) | 15.0 (0.066) | 17.0 (0.14) | | |
| 5—A—4—(a) | $PbZn_{0.4}Th_{0.01}O_x$ | PbO | $Zn(NO_3)_2.6H_2O$ | $Th(NO_3)_4.4H_2O$ | 400/1 | 800/4 |
| | | 80.0 (0.36) | 43.0 (0.14) | 2.0 (0.0036) | | |
| 5—B—1—(a) | $PbY_{0.35}Zr_{0.35}O_x$ | PbO | $Y_2O_3$ | $ZrO_2$ | 400/2 | 700/12 |
| | | 90.0 (0.40) | 15.8 (0.070) | 17.0 (0.14) | | |
| 5—B—2—(a) | $PbHo_{0.17}Zr_{0.35}O_x$ | PbO | $Ho_2O_3$ | $ZrO_2$ | 400/2 | 800/8 |
| | | 90.0 (0.40) | 13.0 (0.034) | 17.0 (0.14) | | |
| 5—B—3—(a) | $PbZn_{0.33}Co_{0.17}O_x$ | PbO | ZnO | $Co(NO_3)_2.6H_2O$ | 450/1 | 800/4 |
| | | 100.0 (0.45) | 12.2 (0.15) | 21.8 (0.075) | | |

Example 6

A. *Toluene Conversion Reactor* — A stainless steel tube 20.32 centimeters (8 inches) in length and 0.95 centimeter (0.375 inch) in internal diameter having a usable capacity of 11 milliliters was employed as a reactor for the toluene conversion reaction. The reactor was arranged vertically and equipped at the upper end with reactant inlet means having calibrated flow controllers and vaporizers, and at the lower end with reaction effluent outlet means for collecting the reaction effluent or, alternatively, for direct introduction thereof via a gas sampling valve into a gas-liquid chromatograph for analysis. The outlet means was also equipped with means for introducing an inert gas diluent — nitrogen or helium, for example — into the reaction effluent for analysis purposes. A radiant furnace was used to maintain a constant temperature during the reaction period. The temperature was measured with a thermocouple in a temperature well located in the lower outside wall of the reactor.

B. *Toluene Conversion* — The reaction was conducted in the stoichiometric mode of operation unless otherwise noted. The reactor was charged with approximately 11 milliliters of the inorganic metal/oxygen composition prepared as described in Examples 1—5 above. Glass wool plugs were used as supports for the composition. The charged reactor was placed in a radiant furnace and heated to maintain a constant temperature throughout the reaction period. Steam and toluene in a 2:1 mole ratio were fed to the reactor at a pressure of $1.013 \times 10^5$ pascal (1 atmosphere) at a rate sufficient to provide a reactor residence (contact) time of 4 seconds (unless otherwise noted) for the toluene (assuming a 50% void space in the reactor). After the reaction had proceeded for 1 minute, the reaction effluent, diluted with helium, was analyzed by gas-liquid chromatography. The results are tabulated in Table 6.

TABLE 6

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 1—A—1—(a) | 630 | 82.7 | 45.7 | 0.3 | 33.0 | 46.0 |
| | 570 | 41.6 | 62.1 | 3.4 | 22.0 | 65.5 |
| | 450 | 3.2 | 15.6 | 27.7 | 31.6 | 43.3 |
| 1—A—1—(b) | 630 | 94.2 | 11.8 | 0.0 | 60.4 | 11.8 |
| | 570 | 45.3 | 52.3 | 0.1 | 33.6 | 52.4 |
| | 540 | 28.6 | 56.2 | 1.6 | 32.3 | 57.8 |
| 1—A—1—(c) | 630 | 86.0 | 33.0 | 0.0 | 43.0 | 33.0 |
| | 570 | 47.6 | 62.2 | 0.4 | 27.0 | 62.6 |
| | 480 | 8.0 | 50.0 | 15.3 | 22.0 | 65.3 |
| 1—A—1—(d) | 630 | 49.3 | 20.7 | 1.9 | 53.8 | 22.6 |
| | 540 | 44.5 | 61.1 | 4.0 | 25.3 | 65.1 |
| | 450 | 3.3 | 15.1 | 25.2 | 41.0 | 40.3 |
| 1—A—1—(e) | 630 | 90.3 | 13.5 | 0.0 | 60.7 | 13.5 |
| | 570 | 35.3 | 43.0 | 0.0 | 41.1 | 43.0 |
| | 480 | 12.3 | 48.1 | 5.3 | 37.0 | 53.4 |
| 1—A—2—(a) | 630 | 24.9 | 65.9 | 8.5 | 12.7 | 74.4 |
| | 570 | 18.0 | 77.2 | 6.2 | 0.3 | 83.5 |
| | 450 | 4.8 | 34.2 | 35.2 | 7.6 | 69.4 |

TABLE 6 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 1—B—1—(a) | 630 | 93.6 | 6.7 | 0.0 | 67.8 | 6.7 |
| | 540 | 31.9 | 60.2 | 0.3 | 30.0 | 60.5 |
| | 450 | 9.9 | 30.9 | 7.2 | 39.3 | 38.1 |
| 1—B—1—(b) | 630 | 96.0 | 4.8 | 0.0 | 70.4 | 4.8 |
| | 540 | 57.6 | 55.7 | 0.0 | 25.7 | 55.7 |
| | 510 | 19.3 | 63.0 | 0.6 | 25.5 | 63.6 |
| | 450 | 12.0 | 57.1 | 1.0 | 25.9 | 58.1 |
| 1—B—1—(c) | 630 | 89.0 | 6.2 | 0.0 | 72.9 | 6.2 |
| | 540 | 27.7 | 53.1 | 0.0 | 39.0 | 53.1 |
| | 450 | 4.5 | 31.5 | 6.3 | 42.3 | 37.8 |
| 1—B—1—(d) | 630 | 72.6 | 20.7 | 0.0 | 60.6 | 20.7 |
| | 540 | 17.3 | 61.7 | 0.1 | 30.8 | 61.8 |
| | 450 | 3.2 | 29.6 | 15.9 | 32.3 | 45.5 |
| 1—B—1—(e) | 630 | 53.7 | 3.1 | 0.0 | 74.2 | 3.1 |
| | 541 | 9.1 | 7.3 | 0.0 | 45.3 | 7.3 |
| | 450 | 2.1 | 0.0(?) | 15.8 | 36.3 | 15.8 |
| 1—B—2—(a) | 630 | 72.8 | 19.3 | 0.0 | 56.9 | 19.3 |
| | 540 | 46.7 | 53.5 | 1.0 | 42.3 | 54.5 |
| | 450 | 6.9 | 50.7 | 9.2 | 25.6 | 59.9 |

TABLE 6 (Cont'd)

| SAMPLE NO. | TEMPERATURE, °C./ CONTACT TIME SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 2—A—1—(a) | 630 | 90.6 | 10.3 | 0.3 | 59.8 | 10.6 |
| | 540 | 19.0 | 56.5 | 3.5 | 30.3 | 60.0 |
| | 480 | 7.1 | 65.0 | 8.0 | 12.1 | 73.0 |
| 2—A—2—(a) | 600 | 85.5 | 2.6 | 0.0 | 89.1 | 2.6 |
| | 540 | 30.0 | 19.6 | 0.1 | 70.3 | 19.7 |
| | 450 | 6.5 | 23.1 | 3.3 | 47.4 | 26.4 |
| 2—B—1—(a) | 630 | 92.0 | 12.0 | 0.0 | 81.5 | 12.0 |
| | 540 | 20.2 | 59.9 | 0.0 | 41.4 | 59.9 |
| | 480 | 3.4 | 57.3 | 1.1 | 29.8 | 58.4 |
| 2—B—1—(b) | 630 | 70.0 | 52.6 | 0.4 | 34.9 | 53.0 |
| | 540 | 14.5 | 80.0 | 1.0 | 17.9 | 81.0 |
| | 480 | 1.9 | 54.6 | 12.4 | 23.5 | 67.0 |
| 2—B—1—(c) | 630 | 85.0 | 22.3 | 0.0 | 70.5 | 22.3 |
| | 540 | 17.6 | 69.1 | 0.0 | 28.8 | 69.1 |
| | 480 | 1.7 | 54.2 | 2.4 | 33.0 | 56.6 |
| 2—B—1—(d) | 630 | 82.1 | 41.4 | 0.0 | 26.3 | 41.4 |
| | 540 | 12.9 | 55.8 | 21.4 | 15.7 | 77.2 |
| | 480 | 1.1 | 10.9 | 22.3 | 38.7 | 33.2 |

TABLE 6 (Cont'd)

| SAMPLE NO. | TEMPERATURE, °C./ CONTACT TIME SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 2—B—1—(e) | 630 | 58.0 | 56.2 | 0.4 | 28.5 | 56.6 |
| | 540 | 10.6 | 70.2 | 6.1 | 19.3 | 76.3 |
| | 480 | 1.5 | 38.3 | 18.9 | 28.3 | 57.2 |
| 2—B—1—(f) | 630 | 69.1 | 49.0 | 0.0 | 37.0 | 49.0 |
| | 540 | 11.0 | 75.5 | 4.2 | 19.9 | 79.7 |
| | 480 | 1.8 | 31.9 | 12.0 | 40.2 | 43.9 |
| 2—B—1—(g) | 630 | 68.4 | 47.4 | 0.0 | 39.9 | 47.4 |
| | 540 | 15.3 | 67.7 | 3.0 | 26.7 | 70.7 |
| | 480 | 3.7 | 45.8 | 11.4 | 30.5 | 57.2 |
| 3—A—1—(a) | 570 | 39.1 | 38.5 | 4.3 | 39.1 | 42.8 |
| 3—B—1—(a) | 570 | 35.3 | 64.3 | 3.1 | 8.5 | 67.4 |
| 4—B—1—(a) | 630 | 60.0 | 56.2 | 3.6 | 24.4 | 59.8 |
| | 570 | 20.0 | 72.1 | 16.0 | 11.0 | 88.1 |
| | 450 | 0.7 | 10.8 | 36.5 | 38.6 | 47.3 |
| 4—B—1—(b) | 630 | 47.7 | 53.7 | 7.3 | 22.0 | 61.1 |
| | 570 | 19.5 | 60.6 | 23.3 | 10.6 | 83.9 |
| | 450 | 0.6 | 6.5 | 32.5 | 40.9 | 39.0 |

TABLE 6 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 4—B—1—(c) | 630 | 51.2 | 36.3 | 7.5 | 26.5 | 43.8 |
| | 570 | 17.8 | 39.7 | 34.5 | 13.3 | 74.2 |
| | 450 | 2.0 | 9.1 | 29.4 | 33.0 | 38.5 |
| 4—B—1—(d) | 630 | 43.6 | 53.2 | 9.5 | 21.2 | 62.7 |
| | 570 | 14.1 | 50.2 | 30.8 | 11.8 | 81.0 |
| | 450 | 0.5 | 0.0 | 23.2 | 45.0 | 23.2 |
| 4—B—1—(e) | 630 | 57.5 | 52.1 | 2.7 | 29.4 | 54.8 |
| | 570 | 29.6 | 66.5 | 7.7 | 18.5 | 74.2 |
| | 450 | 1.3 | 4.8 | 12.2 | 56.7 | 17.0 |
| 5—A—1—(a) | 630 | 71.8 | 57.6 | 2.5 | 20.8 | 60.1 |
| | 570 | 29.2 | 58.9 | 16.3 | 14.5 | 75.2 |
| | 450 | 1.1 | 3.4 | 31.7 | 41.4 | 35.1 |
| 5—A—2—(a) | 630 | 65.9 | 32.6 | 0.0 | 49.7 | 32.6 |
| | 570 | 33.5 | 78.6 | 0.0 | 18.6 | 78.6 |
| | 450 | 2.4 | 13.5 | 29.3 | 11.4 | 42.9 |
| 5—A—3—(a) | 630 | 90.0 | 14.9 | 0.0 | 73.6 | 14.9 |
| | 570 | 45.7 | 54.5 | 0.0 | 36.5 | 54.5 |
| | 450 | 1.9 | 19.9 | 8.1 | 56.0 | 28.0 |

TABLE 6 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 5—A—4—(a) | 630 | 91.1 | 11.0 | 0.0 | 81.3 | 11.0 |
| | 570 | 27.8 | 59.7 | 0.0 | 31.1 | 59.7 |
| | 450 | 0.9 | 19.2 | 2.4 | 45.1 | 21.6 |
| 5—B—1—(a) | 630 | 90.0 | 14.9 | 0.0 | 73.6 | 14.9 |
| | 570 | 45.7 | 54.5 | 0.0 | 36.5 | 54.5 |
| | 450 | 1.9 | 20.1 | 8.0 | 56.0 | 28.1 |
| 5—B—2—(a) | 630 | 80.5 | 33.2 | 0.0 | 56.2 | 33.2 |
| | 570 | 53.7 | 57.8 | 0.0 | 33.0 | 57.8 |
| | 510 | 23.8 | 64.9 | 2.5 | 25.8 | 67.4 |
| | 450 | 3.5 | 27.4 | 18.7 | 41.9 | 46.1 |
| 5—B—3—(a) | 630 | 97.9 | 3.3 | 0.0 | 65.9 | 3.3 |
| | 540 | 51.4 | 47.5 | 0.1 | 35.8 | 47.6 |
| | 450 | 16.1 | 16.2 | 18.1 | 35.3 | 34.3 |

[1]A contact time of 4 seconds was employed unless otherwise noted.

Example 7

*Procedure A* — A series of inorganic metal/oxygen compositions having varied Pb/$M^1$ atomic ratios were prepared by intimately mixing the appropriate amount in grams of lead (II) oxide (PbO) and at least one $M^1$ oxide (or hydroxide) in water, filtering to remove excess water or, alternatively, heating to evaporate the water, and then drying. The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 9 below. The parameters for such compositions, conveniently designated at 7—A—, are set forth in Table 7.

*Procedure B* — A series of inorganic metal/oxygen compositions having varied Pb/$M^1$ atomic ratios and supported on a calcium aluminate cement can be prepared according to the procedure described in Procedure A above except as follows. Following the final calcination, remove the calcined material from the oven, cool, crush, and mix with a calcium aluminate cement (Alcoa—CA—25) in an amount corresponding to 25% by weight of the calcined material. Slurry the solid mixture with water to form a thick paste and allow to air dry in an open casserole dish for 2 hours at 400°C. and then at a final temperature of 700°C. for an additional 2 hours. Remove the supported inorganic metal/oxygen composition from the oven, cool, crush in a mortar, and sieve to 14/30 mesh particles for evaluation in the toluene conversion reactor described in Example 9 below. Following these steps should result in compositions, conveniently designated as 7—B—, having properties beneficial for use in the process of this invention.

TABLE 7

| SAMPLE NO. | $PbM^1_aO_x$ | STARTING MATERIALS GRAMS (MOLES) Pb | M¹ | ATOMIC RATIO Pb/M¹ | CALCINATION CONDTIONS TEMPERATURE, °C./TIME HOURS INITIAL | FINAL |
|---|---|---|---|---|---|---|
| 7—A—1— | $PbSr_aO_x$ | PbO | SrO | Pb/Sr | | |
| (a) | $PbSr_{0.1}Ox$ | 145.0 (0.65) | 6.7 (0.065) | 10 | 450/1 | 800/16 |
| (b) | $PbSr_{0.33}O_x$ | 130.0 (0.58) | 20.0 (0.19) | 3 | 450/1 | 800/16 |
| (c) | $PbSr_{0.5}O_x$ | 120.0 (0.54) | 28.0 (0.27) | 2 | (1) 450/1 | (2) 800/16 |
| | | | | | | (3) 900/12 |
| (d) | $PbSrO_x$ | 60.0 (0.27) | 28.0 (0.27) | 1 | 450/1 | 800/16 |
| (e) | $PbSr_2O_x$ | 80.0 (0.36) | 74.3 (0.72) | 0.5 | 450/1 | 800/16 |
| (f) | $PbSr_3O_x$ | 65.0 (0.29) | 91.0 (0.88) | 0.33 | 450/1 | 800/16 |
| 7—A—2— | $PbMg_aO_x$ | PbO | MgO | Pb/Mg | | |
| (a) | $PbMgO_x$ | 89.3 (0.40) | 16.0 (0.40) | 1 | 450/1 | 750/8 |
| 7—A—3— | $PbBa_aO_x$ | PbO | BaO | Pb/Ba | | |
| (a) | $PbBaO_x$ | 44.6 (0.20) | 30.6 (0.20) | 1 | 450/1 | 750/8 |
| 7—A—4— | $PbTl_aO_x$ | PbO | $Tl_2O_3$ | Pb/Tl | | |
| (a) | $PbTl_2O_x$ | 33.5 (0.15) | 68.4 (0.15) | 0.5 | 350/2 | 700/6 |

Example 8

A series of inorganic metal/oxygen compositions containing two or more $M^1$ elements were prepared by intimately mixing the appropriate amount of lead (II) oxide (PbO), lead (II) carbonate ($PbCO_3$), or lead (II) nitrate [$Pb(NO_3)_2$] with at least two $M^1$ carbonates, nitrates, or oxides (or, alternatively, mixing an appropriate amount of a suitable lead-containing binary metal/oxygen composition with at least one $M^1$ carbonate, nitrate, or oxide) in water and heating to evaporate the water and/or decompose the carbonates and/or nitrates. The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 9 below. The parameters for such compositions, conveniently designated as 8—A—, are set forth in Table 8.

TABLE 8

| | | STARTING MATERIALS GRAMS (MOLES) | | | CALCINATION CONDITIONS TEMPERATURE, °C./TIME, HOURS | |
|---|---|---|---|---|---|---|
| SAMPLE NO. | $PbM^1_aO_x$ | Pb | $M^1$ | | INITIAL | FINAL |
| 8—A—1—(a) | $PbMg_3Sr_{0.5}O_x$ | PbO | MgO | SrO | | |
| | | 111.6 (0.50) | 58.9 (1.47) | 25.9 (0.25) | 450/1 | 850/8 |

Example 9

A. *Toluene Conversion Reactor* — A stainless steel tube 20.32 centimeters (8 inches) in length and 0.95 centimeter (0.375 inch) in internal diameter having a usable capacity of 11 milliliters was employed as a reactor for the toluene conversion reaction. The reactor was arranged vertically and equipped at the upper end with reactant inlet means having calibrated flow controllers and vaporizers, and at the lower end with reaction effluent outlet means for collecting the reaction effluent or, alternatively, for direct introduction thereof via a gas sampling valve into a gas-liquid chromatograph for analysis. The outlet means was also equipped with means for introducing an inert gas diluent — nitrogen or helium, for example — into the reaction effluent for analysis purposes. A radiant furnace was used to maintain a constant temperature during the reaction period. The temperature was measured with a thermocouple in a temperature well located on the lower outside wall of the reactor.

B. *Toluene Conversion* — The reaction was conducted in the stoichiometric mode of operation unless otherwise noted. The reactor was charged with approximately 11 milliliters of the inorganic metal/oxygen composition prepared as described in Examples 7 and 8 above. Glass wool plugs were used as supports for the composition. The charged reactor was placed in a radiant furnace and heated to maintain a constant temperature throughout the reaction period. Steam and toluene in a 2:1 mole ratio were fed to the reactor at a pressure of $1.013 \times 10^5$ pascal (1 atmosphere) at a rate sufficient to provide a reactor residence (contact) time of 4 seconds (unless otherwise noted) for the toluene (assuming a 50% void space in the reactor). After the reaction had proceeded for 1 minute, the reaction effluent, diluted with helium, was analyzed by gas-liquid chromatography. The results are tabulated in Table 9.

TABLE 9

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 7—A—1—(a) | 630 | 74.4 | 45.2 | 0.5 | 36.2 | 45.7 |
| | 570 | 37.0 | 58.8 | 2.8 | 22.8 | 61.6 |
| | 450 | 5.2 | 15.1 | 24.7 | 14.3 | 39.8 |
| 7—A—1—(b) | 630 | 78.2 | 18.9 | 2.0 | 45.4 | 20.9 |
| | 540 | 33.7 | 39.8 | 16.3 | 38.9 | 56.1 |
| | 480 | 5.3 | 9.1 | 21.6 | 18.3 | 30.7 |
| 7—A—1—(c) | 630 | 48.7 | 26.0 | 6.3 | 34.9 | 32.3 |
| | 540 | 19.9 | 31.6 | 15.5 | 22.6 | 47.1 |
| | 480 | 2.0 | 3.3 | 18.0 | 15.7 | 21.3 |
| 7—A—1—(d) | 630 | 97.5 | 2.8 | 0.3 | 89.8 | 3.1 |
| | 570 | 53.1 | 57.0 | 8.7 | 33.1 | 55.7 |
| | 510 | 21.0 | 51.1 | 20.5 | 22.3 | 71.6 |
| | 450 | 5.9 | 31.1 | 39.9 | 23.3 | 71.0 |
| 7—A—1—(e) | 630 | 73.3 | 30.8 | 5.2 | 46.0 | 36.0 |
| | 540 | 44.4 | 45.3 | 14.3 | 32.8 | 59.6 |
| | 480 | 9.2 | 37.2 | 38.1 | 20.2 | 75.3 |
| 7—A—1—(f) | 630 | 63.8 | 16.5 | 6.5 | 40.4 | 23.0 |
| | 540 | 19.0 | 37.0 | 37.1 | 14.7 | 74.1 |
| | 480 | 4.7 | 12.2 | 64.0 | 15.8 | 76.2 |

TABLE 9 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 7—A—2—(a) | 630 | 89.2 | 16.7 | 0.3 | 52.7 | 17.0 |
| | 540 | 44.0 | 56.0 | 0.8 | 31.6 | 56.8 |
| | 450 | 2.4 | 38.5 | 25.8 | 17.3 | 64.3 |
| 7—A—3—(a) | 630 | 91.5 | 16.3 | 0.4 | 62.2 | 16.7 |
| | 540 | 52.7 | 32.3 | 7.3 | 50.1 | 39.6 |
| 7—A—4—(a) | 480 | 10.7 | 17.0 | 43.4 | 3.8 | 60.4 |
| 8—A—1—(a) | 630 | 39.5 | 29.2 | 25.6 | 19.0 | 54.8 |
| | 575 | 29.8 | 32.5 | 37.2 | 13.9 | 69.7 |
| | 500 | 4.9 | 9.8 | 62.7 | 7.1 | 72.5 |

[1]A contact time of 4 seconds was employed unless otherwise noted.

Example 10

*Procedure A* — A series of inorganic metal/oxygen compositions were prepared by intimately mixing the appropriate amount of lead(II) oxide (PbO), lead(II) carbonate ($PbCO_3$), or lead(II) nitrate [$Pb(NO_3)_2$] with two $M^1$ carbonates, nitrates, or oxides, [or, alternatively, mixing an appropriate amount of a suitable lead-containing binary metal/oxygen composition with at least one additional $M^1$ carbonate, nitrate, or oxide, depending on the element other than lead contained in the binary composition] in water and heating to evaporate the water (and decompose the carbonates and/or nitrates, when employed). The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 11 below. The parameters for such compositions, conveniently designated as 10—A—, are set forth in Table 10.

*Procedure B* — A series of inorganic metal/oxygen compositions supported on a calcium aluminate cement can be prepared according to the procedure described in Procedure A above except as follows. Following the final calcination, remove the calcined material from the oven, cool, crush, and mix with a calcium aluminate cement (Alcoa—CA—25) in an amount corresponding to 25% by weight of the calcined material. Slurry the solid mixture with water to form a thick paste and allow to air dry. Calcine the air-dried paste in air in an open casserole dish for 2 hours at 400°C. and then at a final temperature of 700°C. for an additional 2 hours. Remove the supported inorganic metal/oxygen composition from the oven, cool, crush in a mortar, and sieve to 14/30 mesh particles for evaluation in the toluene conversion reactor described in Example 11 below. Following these steps should result in compositions, conveniently designated as 10—B—, having properties beneficial for use in the process of this invention.

TABLE 10

| SAMPLE NO. | $PbM^1_aO_x$ | STARTING MATERIALS GRAMS (MOLES) | | | CALCINATION CONDITIONS TEMPERATURE, °C./TIME HOURS | |
|---|---|---|---|---|---|---|
| | | Pb | $M^1$ | | INITIAL | FINAL |
| 10—A—1—(a) | $PbMg_{0.25}Y_{0.5}O_x$ | PbO | MgO | $Y_2O_3$ | | |
| | | 89.3 (0.40) | 4.0 (0.10) | 22.6 (0.10) | 450/1 | 1000/64 |
| 10—A—2—(a) | $PbSr_{0.67}K_{0.67}O_x$ | PbO | SrO | 85% KOH | | |
| | | 133.9 (0.60) | 41.4 (0.40) | 26.4 (0.40) | 450/1 | 850/8 |
| 10—A—3—(a) | $PbSrZr_{0.1}O_x$ | $PbZrO_x$[1] | SrO | — | | |
| | | 100.0 (0.29) | 3.0 (0.029) | | 450/1 | 800/4 |

[1]The lead zirconate binary metal/oxygen composition having a (calculated gram molecular weight of 346.4 grams, was prepared as follows:

Lead(II) oxide (PbO; 70.0 grams, 0.31 mole) and zirconium(IV) oxide ($ZrO_2$; 38.7 grams, 0.31 mole) were intimately mixed in water and then heated to evaporate the water. The resulting solid was placed in an open casserole dish and calcined in air for 2 hours at 400°C. And then at 700°C. for an additional 12 hours. The calcined material was removed from the oven, cooled, crushed in a mortar, and ground to a fine powder.

Example 11

A. *Toluene Conversion Reactor* — A stainless steel tube 20.32 centimeters (8 inches) in length and 0.95 centimeter (0.375 inch) in internal diameter having a usable capacity of 11 milliliters was employed as a reactor fro the toluene conversion reaction. The reactor was arranged vertically and equipped at the upper end with reactant inlet means having calibrated flow controllers and vaporizers, and at the lower end with reaction effluent outlet means for collecting the reaction effluent or, alternatively, for direct introduction thereof via a gas sampling valve into a gas-liquid chromatograph for analysis. The outlet means was also equipped with means for introducing an inert gas diluent — nitrogen or helium, for example — into the reaction of effluent for analysis purposes. A radiant furnace was used to maintain a constant temperature during the reaction period. The temperature was measured with a thermocouple in a temperature well located on the lower outside wall of the reactor.

B. *Toluene Conversion* — The reaction was conducted in the stoichiometric mode of operation unless otherwise noted. The reactor was charged with approximately 11 milliliters of the inorganic metal/oxygen composition prepared as described in Example 10 above. Glass wool plugs were used as supports for the composition. The charged reactor was placed in a radiant furnace and heated to maintain a constant temperature throughout the reaction period. Steam and toluene in a 2:1 mole ratio were fed to the reactor at a pressure of $1.013 \times 10^5$ pascal (1 atmosphere) at a rate sufficient to provide a reactor residence (contact) time of 4 seconds (unless otherwise noted) for the toluene (assuming a 50% void space in the reactor). After the reaction had proceeded for 1 minute, the reaction effluent, diluted with helium, was analyzed by gas-liquid chromatography. The results are tabulated in Table 11.

TABLE 11

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 10—A—1—(a) | 630 | 68.8 | 45.9 | 0.0 | 55.2 | 45.9 |
| | 575 | 33.8 | 65.7 | 1.1 | 33.8 | 66.8 |
| | 500 | 15.1 | 48.2 | 6.0 | 49.6 | 54.2 |
| 10—A—2—(a) | 630 | 44.6 | 23.2 | 5.3 | 66.5 | 28.5 |
| | 575 | 43.5 | 66.0 | 6.6 | 21.6 | 72.6 |
| | 500 | 2.5 | 13.0 | 39.7 | 26.1 | 52.7 |
| 10—A—3—(a) | 630 | 55.5 | 65.2 | 1.3 | 31.7 | 66.5 |
| | 575 | 40.0 | 73.6 | 1.6 | 24.7 | 75.2 |
| | 500 | 9.7 | 35.4 | 11.4 | 13.1 | 46.8 |

[1]A contact time of 4 seconds was employed unless otherwise noted.

Example 12

*Procedure A* — A series of inorganic metal/oxygen compositions having varied Bi/$M^3$ atomic ratios were prepared by intimately mixing the appropriate amount in grams of bismuth (III) oxide ($Bi_2O_3$) and at least one $M^3$ oxide (or hydroxide) in water, filtering to remove excess water or, alternatively, heating to evaporate the water, and then drying. The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 15 below. The parameters for such compositions, conveniently designated as 12—A—, are set forth in Table 12.

*Procedure B* — The procedure described in Procedure A above was employed except that following the final calcination, the calcined material was removed from the oven, cooled, crushed, and mixed with a calcium aluminate cement (Alcoa—CA—25) in an amount corresponding to 25% by weight of the dry weight of the calcined material. The resulting solid mixture was slurried with water to form a thick paste and allowed to air dry. The air-dried paste was calcined in air in an open casserole dish for 2 hours at 400°C. and then at a final temperature of 700°C. for an additional 2 hours. The supported inorganic metal/oxygen composition was removed from the oven, cooled, crushed in a mortar, and sieved to 14/30 mesh particles for evaluation in the toluene conversion reactor described in Example 15 below. The parameters for such compositions, conveniently designated as 12—B—, are set forth in Table 12.

TABLE 12

| SAMPLE NO. | $BiM^3_aO_x$ | STARTING MATERIALS GRAMS (MOLES) Bi | M³ | ATOMIC RATIO Bi/M³ | CALCINATION CONDITIONS TEMPERATURE, °C./TIME, HOURS INITIAL | FINAL |
|---|---|---|---|---|---|---|
| 12—A—1— | $BiZn_aO_x$ | $Bi_2O_3$ | ZnO | Bi/Zn | | |
| (a) | $BiZn_{0.11}O_x$ | 150.0 (0.32) | 5.8 (0.071) | 9 | (1) 400/2 | (3) 800/1 |
| | | | | | (2) 720/8 | (4) 730/1 |
| (b) | $BiZn_{0.17}O_x$ | 150.0 (0.32) | 8.9 (0.11) | 6 | (1) 400/2 | (3) 800/1 |
| | | | | | (2) 720/8 | (4) 730/1 |
| (c) | $BiZn_{0.25}O_x$ | 140.0 (0.30) | 12.2 (0.15) | 4 | (1) 400/2 | (2) 800/1 |
| | | | | | | (3) 730/1 |
| (d) | $BiZn_{0.5}$ | 130.0 (0.28) | 22.7 (0.28) | 2 | (1) 400/2 | (3) 800/1 |
| | | | | | (2) 700/2 | (4) 730/1 |
| (e) | $BiZnO_x$ | 110.0 (0.24) | 38.4 (0.47) | 1 | (1) 400/2 | (3) 800/1 |
| | | | | | (2) 700/8 | (4) 730/1 |
| (f) | $BiZn_2O_x$ | 90.0 (0.19) | 63.0 (0.77) | 0.5 | (1) 400/2 | (3) 800/1 |
| | | | | | (2) 700/2 | (4) 730/1 |
| (g) | $BiZn_3O_x$ | 70.0 (0.15) | 73.0 (0.90) | 0.33 | (1) 400/2 | (3) 800/1 |
| | | | | | (2) 700/8 | (4) 730/1 |

TABLE 12 (Cont'd)

| SAMPLE NO. | $BiM^3_aO_x$ | STARTING MATERIALS GRAMS (MOLES) Bi | M³ | ATOMIC RATIO Bi/M³ | CALCINATION CONDITIONS TEMPERATURE, °C./TIME, HOURS INITIAL | FINAL |
|---|---|---|---|---|---|---|
| 12—A—2 | $BiK_aO_x$ | $Bi_2O_3$ | 85% KOH | Bi/K | | |
| (a) | $BiK_{0.013}O_x$ | 100.0 (0.21) | 0.35 (0.0053) | 79 | — | 750/8 |
| (b) | $BiK_{0.21}O_x$ | 100.0 (0.21) | 5.8 (0.088) | 4.8 | — | 750/8 |
| (c) | $BiK_{0.43}O_x$ | 100.0 (0.21) | 11.6 (0.18) | 2.3 | — | 850/8 |
| (d) | BiKO | 96.8 (0.21) | 26.4 (0.40) | .1 | — | 750/8 |
| 12—A—3— | $BiNi_aO_x$ | $Bi_2O_3$ | NiO | Bi/Ni | | |
| (a) | $BiNi_{0.2}O_x$ | 140.0 (0.30) | 9.0 (0.12) | 5 | 450/2 | 800/6 |
| (b) | $BiNi_{0.33}O_x$ | 135.0 (0.29) | 14.4 (0.19) | 3 | 450/2 | 800/6 |
| (c) | $BiNi_{0.5}O_x$ | 140.0 (0.30) | 22.4 (0.30) | 2 | 450/2 | 800/6 |
| (d) | $BiNiO_x$ | 110.0 (0.24) | 35.3 (0.47) | 1 | (1) 450/2 | (2) 800/6<br>(3) 1000/12 |
| (e) | $BiNi_2O_x$ | 90.0 (0.19) | 57.7 (0.77) | 0.5 | (1) 450/2 | (2) 800/6<br>(3) 1000/12 |
| (f) | $BiNi_3O_x$ | 80.0 (0.17) | 76.9 (1.03) | 0.33 | (1) 450/2 | (2) 800/6<br>(3) 1000/12 |

TABLE 12 (Cont'd)

| SAMPLE NO. | $BiM^3_aO_x$ | STARTING MATERIALS GRAMS (MOLES) Bi | M³ | ATOMIC RATIO Bi/M³ | CALCINATION CONDITIONS TEMPERATURE, °C./TIME, HOURS INITIAL | FINAL |
|---|---|---|---|---|---|---|
| 12—A—4— | $BiZr_aO_x$ | $Bi_2O_3$ | $ZrO_2$ | Bi/Zr | | |
| (a) | $BiZr_{0.17}O_x$ | 139.8 (0.30) | 12.3 (0.10) | 6 | 450/1 | 900/8 |
| (b) | $BiZr_{0.33}O_x$ | 139.8 (0.30) | 24.6 (0.20) | 3 | 450/1 | 900/8 |
| (c) | $BiZrO_x$ | 93.2 (0.20) | 49.2 (0.40) | 1 | 450/1 | 900/8 |
| (d) | $BiZr_2O_x$ | 93.2 (0.20) | 98.6 (0.80) | 0.5 | 450/1 | 900/8 |
| (e) | $BiZr_3O_x$ | 46.6 (0.10) | 73.8 (0.60) | 0.33 | 450/1 | 900/8 |
| 12—A—5— | $BiGe_aO_x$ | $Bi_2O_3$ | $GeO_2$ | Bi/Ge | | |
| (a) | $BiGe_{0.17}O_x$ | 116.6 (0.25) | 8.9 (0.085) | 6 | 450/1 | 850/8 |
| (b) | $BiGe_{0.7}O_x$ | 46.6 (0.10) | 35.5 (0.34) | 0.6 | 450/1 | 850/8 |
| (c) | $BiGe_{3.8}O_x$ | 61.7 (0.13) | 104.5 (1.00) | 0/26 | 450/1 | 800/10 |
| 12—A—6— | $BiCe_aO_x$ | $Bi_2O_3$ | $CeO_2$ | Bi/Ce | | |
| (a) | $BiCe_{0.17}O_x$ | 139.8 (0.30) | 17.2 (0.10) | 6 | 450/1 | 850/8 |
| (b) | $BiCe_{0.33}O_x$ | 139.8 (0.30) | 34.4 (0.20) | 3 | 450/1 | 850/8 |
| (c) | $BiCe_{0.5}O_x$ | 116.5 (0.25) | 43.0 (0.25) | 2 | 450/1 | 850/8 |
| (d) | $BiCeO_x$ | 93.2 (0.20) | 68.8 (0.40) | 1 | 450/1 | 850/8 |
| (e) | $BiCe_2O_x$ | 46.6 (0.10) | 68.8 (0.40) | 0.5 | 450/1 | 850/8 |
| (f) | $BiCe_3O_x$ | 46.6 (0.10) | 103.2 (0.60) | 0.33 | 450/1 | 850/8 |

TABLE 12 (Cont'd)

| SAMPLE NO. | $BiM^3_aO_x$ | STARTING MATERIALS GRAMS (MOLES) BI | M³ | ATOMIC RATIO $Bi/M^3$ | CALCINATION CONDITIONS TEMPERATURE, °C./TIME, HOURS INITIAL | FINAL |
|---|---|---|---|---|---|---|
| 12—B—1— | $BiLa_aO_x$ | $Bi_2O_3$ | $La_2O_3$ | Bi/La | | |
| (a) | $BiLa_{0.33}O_x$ | 140.0 (0.30) | 31.4 (0.10) | 3 | — | 1000/5 |
| (b) | $BiLa_{0.5}O_x$ | 120.0 (0.26) | 40.4 (0.12) | 2 | — | 1000/5 |
| (c) | $BiLaO_x$ | 96.8 (0.21) | 65.2 (0.20) | 1 | — | 1000/5 |
| (d) | $BiLa_2O_x$ | 70.0 (0.15) | 94.2 (0.29) | 0.5 | — | 1000/5 |
| (e) | $BiLa_3O_x$ | 55.0 (0.12) | 111.0 (0.34) | 0.35 | — | 1000/5 |

Example 13

*Procedure A* — A series of inorganic metal/oxygen compositions having varied Bi/$M^3$ atomic ratios can be prepared by the following described procedure. Dissolve the appropriate amount in grams of bismuth (III) oxide ($Bi_2O_3$) and at least one $M^3$ nitrate in approximately 100 milliliters of concentrated nitric acid ($HNO_3$) and heat to evaporate and decompose the nitric acid and nitrates. Place the resulting solid in an open casserole dish and calcine in air for 8 hours at 850°C. Remove the calcined material from the oven, cool, crush in a mortar, and sieve to 14/30 mesh particles for evaluation in the toluene conversion reactor described in Example 15 below. Following these steps should result in composition, conveniently designated as 13—A—, having properties beneficial for use in the process of this invention.

*Procedure B* — The procedure described in Procedure A above was employed except that following the final calcination, the calcined material was supported on a calcium aluminate cement (Alcoa—CA—25) as described in Example 12, Procedure B hereinabove. The parameters for such compositions, conveniently designated as 13—B—, are set forth in Table 13.

TABLE 13

| SAMPLE NO. | $BiM^3_aO_x$ | STARTING MATERIALS GRAMS (MOLES) | | ATOMIC RATIO | CALCINATION CONDITIONS TEMPERATURE, °C./TIME, HOURS | |
|---|---|---|---|---|---|---|
| | | Bi | $M^3$ | $Bi/M^3$ | INITIAL | FINAL |
| 13—B—1— | $BiTh_aO_x$ | $Bi(NO_3)_3.5H_2O$ | $Th(NO_3)_4.4H_2O$ | Bi/Th | | |
| (a) | $BiTh_{0.33}O_x$ | 140.0 (0.29) | 53.0 (0.10) | 3 | — | 850/8 |
| (b) | $BiTh_{0.5}O_x$ | 125.0 (0.26) | 71.0 (0.13) | 2 | — | 850/8 |
| (c) | $BiThO_x$ | 90.0 (0.19) | 102.0 (0.18) | 1 | — | 850/8 |
| (d) | $BiTh_2O_x$ | 57.0 (0.12) | 130.0 (0.24) | 0.5 | — | 850/8 |
| (e) | $BiTh_3O_x$ | 44.0 (0.091) | 150 (0.27) | 0.34 | — | 850/8 |

Example 14

A series of inorganic metal/oxygen compositions containing two or more $M^3$ elements were prepared by intimately mixing the appropriate amount of bismuth (III) oxide ($Bi_2O_3$) or bismuth (III) nitrate pentahydrate [$Bi(NO_3)_3.5H_2O$] with at least two $M^3$ nitrates or oxides (or, alternatively, mixing an appropriate amount of a suitable bismuth-containing binary inorganic metal/oxygen composition with at least one additional $M^3$ nitrate or oxide) in water and heating to evaporate the water and/or decompose the nitrates. The resulting solid was placed in an open casserole dish and calcined in air for an initial period at an initial temperature and than at a final temperature for an additional period. The calcined material was removed from the oven, cooled, crushed in a mortar, and sieved to 14/30 mesh particles for evaluation in the toluene conversion reactor described in Example 15 below. The parameters for such compositions, conveniently designated as 14—A—, are set forth in Table 14.

TABLE 14

| SAMPLE NO. | $BiM^3_aO_x$ | STARTING MATERIALS GRAMS (MOLES) Bi | | $M^3$ | CALCINATION CONDITIONS TEMPERATURE, °C./TIME HOURS INITIAL | FINAL |
|---|---|---|---|---|---|---|
| 14—A—1—(a) | $BiTh_{0.16}Zn_{0.47}O_x$ | $Bi_2O_3$ | $Th(NO_3)4.H_2O$ | ZnO | | |
| | | 89.3 (0.19) | 33.1 (0.06) | 14.7 (0.18) | 400/1 | 800/64 |
| 14—A—2—(a) | $BiLaK_{0.5}O_x$ | $Bi_2O_3$ | $La_2O_3$ | 85% KOH | | |
| | | 93.2 (0.20) | 65.2 (0.20) | 13.2 (0.20) | 450/1 | 800/8 |
| 14—A—3—(a) | $BiGe_{0.25}Ni_{0.5}O_x$ | $Bi_2O_3$ | $GeO_2$ | NiO | | |
| | | 93.2 (0.20) | 10.5 (0.10) | 14.9 (0.20) | 450/1 | 800/8 |
| 14—A—4—(a) | $BiZnZrO_x$ | $Bi_2O_3$ | ZnO | $ZrO_2$ | | |
| | | 46.6 (0.10) | 16.3 (0.20) | 24.6 (0.20) | 450/1 | 800/8 |
| 14—A—5—(a) | $BiCe_{0.20}ThO_x$ | $BiThO_x$[1] | $GeO_2$ | — | | |
| | | 85.0 (0.086) | 2.9 (0.017) | | 450/1 | 800/8 |

[1]Prepared according to the procedure described in Example 13, Procedure A, using the quantities of material specified in 13—B—1—(c); based on a (calculated) gram molecular weight of 994.0 grams.

Example 15

A. *Toluene Conversion Reactor* — A stainless steel tube 20.32 centimeters (8 inches) in length and 0.95 centimeter (0.375 inch) in internal diameter having a usable capacity of 11 milliliters was employed as a reactor for the toluene conversion reaction. The reactor was arranged vertically and equipped at the upper end with reactant inlet means having calibrated flow controllers and vaporizers, and at the lower end with reaction effluent outlet means for collecting the reaction effluent or, alternatively, for direct introduction thereof via a gas sampling valve into a gas-liquid chromatograph for analysis. The outlet means was also equipped with means for introducing an inert gas diluent — nitrogen or helium, for example — into the reaction effluent for analysis purposes. A radiant furnace was used to maintain a constant temperature during the reaction period. The temperature was measured with a thermocouple in a temperature well located on the lower outside wall of the reactor.

B. *Toluene Conversion* — The reaction was conducted in the stoichiometric mode of operation unless otherwise noted. The reactor was charged with approximately 11 milliliters of the inorganic metal/oxygen composition prepared as described in Examples 12—14 above. Glass wool plugs were used as supports for the composition. The charged reactor was placed in a radiant furnace and heated to maintain a constant temperature throughout the reaction period. Steam and toluene in a 2:1 mole ratio were fed to the reactor at a pressure of $1.013 \times 10^5$ pascal (1 atomsphere) at a rate sufficient to provide a reactor residence (contact) time of 4 seconds (unless otherwise noted) for the toluene (assuming a 50% void space in the reactor). After the reaction had proceeded for 1 minute, the reaction effluent, diluted with helium, was analyzed by gas-liquid chromatography. The results are tabulated in Table 15.

TABLE 15

| SAMPLE NO. | TEMPERATURE, °C./ CONTACT TIME, SECONDS[1] | CONVERSION % | SELECTIVITY, % | | | |
|---|---|---|---|---|---|---|
| | | | STILBENE | BIBENZYL | BENZENE | STILBENE + BIBENZYL |
| 12—A—1—(a) | 630 | 39.0 | 31.1 | 14.5 | 25.7 | 45.6 |
| | 540 | 12.6 | 42.5 | 34.9 | 12.3 | 77.4 |
| | 510 | 6.8 | 25.2 | 37.7 | 18.7 | 62.9 |
| 12—A—1—(b) | 630 | 53.4 | 26.4 | 5.9 | 36.2 | 32.3 |
| | 540 | 16.7 | 43.8 | 20.6 | 22.0 | 64.4 |
| | 510 | 10.0 | 37.3 | 23.6 | 23.9 | 60.9 |
| 12—A—1—(c) | 630 | 36.4 | 22.5 | 15.9 | 24.6 | 38.4 |
| | 540 | 9.4 | 34.8 | 44.0 | 10.6 | 78.8 |
| | 510 | 4.0 | 15.9 | 51.6 | 15.2 | 67.5 |
| 12—A—1—(d) | 630 | 38.0 | 30.8 | 13.2 | 25.3 | 44.0 |
| | 540 | 11.9 | 45.0 | 30.5 | 13.1 | 75.5 |
| | 510 | 8.6 | 36.4 | 33.5 | 15.8 | 69.9 |
| 12—A—1—(e) | 630 | 46.6 | 31.3 | 8.4 | 29.8 | 39.7 |
| | 540 | 14.6 | 39.4 | 22.3 | 25.3 | 61.7 |
| | 510 | 7.6 | 24.8 | 28.8 | 28.4 | 53.6 |
| 12—A—1—(f) | 630 | 49.0 | 32.0 | 9.2 | 27.8 | 41.2 |
| | 540 | 13.3 | 45.4 | 29.6 | 14.3 | 75.0 |
| | 510 | 6.6 | 27.1 | 39.5 | 18.5 | 66.6 |

TABLE 15 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 12—A—1—(g) | 630 | 48.8 | 31.7 | 7.6 | 29.9 | 39.3 |
| | 540 | 21.5 | 42.3 | 16.2 | 25.7 | 58.5 |
| | 510 | 10.0 | 31.9 | 24.6 | 26.4 | 56.5 |
| 12—A—2—(a) | 630 | 47.3 | 39.4 | 13.6 | 22.2 | 53.0 |
| | 575 | 26.3 | 50.2 | 31.2 | 7.6 | 81.4 |
| 12—A—2—(b) | 630 | 15.9[2] | 41.3 | 20.9 | 28.9 | 62.2 |
| | 575 | 14.9 | 54.2 | 29.3 | 15.4 | 83.5 |
| 12—A—2—(c) | 575 | 38.4[2] | 72.7 | 13.4 | 9.5 | 86.1 |
| | 530 | 14.4[2] | 56.7 | 6.2 | 32.7 | 62.9 |
| | 500 | 1.7[2] | 7.4 | 64.1 | 21.8 | 71.5 |
| 12—A—2—(d) | 575 | 46.0 | 81.3 | 4.8 | 8.7 | 86.1 |
| | 500 | 8.1[2] | 41.6 | 34.3 | 7.2 | 75.8 |
| | 500 | 2.9 | 22.8 | 34.5 | 16.6 | 57.3 |
| 12—A—3—(a) | 630 | 94.8 | 4.5 | 0.4 | 44.0 | 4.9 |
| | 540 | 38.6 | 50.0 | 6.4 | 21.6 | 56.4 |
| | 450 | 4.6 | 3.9 | 1.0 | 29.4 | 4.9 |
| 12—A—3—(b) | 630 | 40.8 | 26.6 | 12.9 | 24.2 | 39.5 |
| | 540 | 11.6 | 41.5 | 38.5 | 9.2 | 80.0 |
| | 450 | 0.9 | 0.0 | 27.3 | 32.0 | 27.3 |

TABLE 15 (Cont'd)

| SAMPLE NO. | TEMPERATURE, °C./ CONTACT TIME, SECONDS[1] | CONVERSION, % | SELECTIVITY, % STILBENE | SELECTIVITY, % BIBENZYL | SELECTIVITY, % BENZENE | SELECTIVITY, % STILBENE + BIBENZYL |
|---|---|---|---|---|---|---|
| 12—A—3—(c) | 630 | 65.9 | 23.2 | 4.7 | 25.2 | 27.9 |
| | 540 | 17.2 | 47.5 | 30.4 | 10.2 | 77.9 |
| | 450 | 1.6 | 9.9 | 43.5 | 20.1 | 53.4 |
| 12—A—3—(d) | 630 | 15.0 | 24.1 | 29.9 | 19.2 | 54.0 |
| | 540 | 6.2 | 18.1 | 49.9 | 13.7 | 68.0 |
| | 450 | 0.3 | 0.0 | 57.9 | 23.0 | 57.9 |
| 12—A—3—(e) | 630 | 18.4 | 30.5 | 29.6 | 17.2 | 60.1 |
| | 540 | 7.8 | 26.1 | 48.2 | 11.1 | 74.3 |
| | 450 | 0.4 | 0.0 | 52.3 | 34.3 | 52.3 |
| 12—A—3—(f) | 630 | 21.3 | 36.1 | 24.1 | 17.0 | 60.2 |
| | 540 | 9.2 | 30.7 | 39.2 | 13.9 | 69.9 |
| | 450 | 0.4 | 0.0 | 47.1 | 31.8 | 47.1 |
| 12—A—4—(a) | 600/2 | 58.8 | 28.4 | 2.2 | 41.7 | 30.6 |
| | 530/2 | 16.0 | 45.9 | 13.7 | 22.6 | 59.6 |
| | 500/2 | 6.7 | 29.2 | 25.7 | 25.4 | 54.9 |
| 12—A—4—(b) | 630/2 | 53.1 | 35.2 | 6.6 | 45.0 | 41.8 |
| | 530/2 | 14.6 | 64.2 | 20.5 | 12.6 | 84.7 |
| | 500/2 | 5.1 | 40.7 | 38.7 | 17.1 | 79.4 |

TABLE 15 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 12—A—4—(c) | 630/2 | 47.9 | 47.0 | 10.5 | 27.7 | 57.5 |
| | 530 | 10.3 | 41.3 | 42.4 | 12.3 | 83.7 |
| | 500/2 | 4.8 | 26.4 | 52.8 | 15.2 | 79.2 |
| 12—A—4—(d) | 603 | 75.2 | 19.5 | 1.8 | 45.5 | 21.3 |
| | 530 | 20.2 | 49.3 | 20.8 | 13.7 | 70.1 |
| | 500 | 8.9 | 27.5 | 35.6 | 14.8 | 63.1 |
| 12—A—4—(e) | 630 | 55.3 | 35.4 | 6.1 | 43.6 | 41.5 |
| | 530 | 24.1 | 58.8 | 16.8 | 19.5 | 75.6 |
| | 500 | 16.4 | 50.0 | 19.2 | 24.4 | 69.2 |
| 12—A—5—(a) | 630 | 44.8 | 18.5 | 8.8 | 55.5 | 27.3 |
| | 530 | 14.9 | 42.1 | 35.7 | 16.5 | 77.8 |
| | 500 | 9.5 | 37.5 | 42.3 | 16.2 | 79.8 |
| 12—A—5—(b) | 630 | 5.5 | 7.4 | 14.8 | 51.7 | 22.2 |
| | 530 | 3.1 | 12.8 | 57.2 | 24.8 | 70.0 |
| | 500 | 2.6 | 15.6 | 58.6 | 19.5 | 74.2 |
| 12—A—5—(c) | 630 | 58.0 | 16.0 | 5.5 | 58.6 | 21.5 |
| | 575 | 24.0 | 30.1 | 9.9 | 42.8 | 40.0 |
| | 500 | 10.4 | 23.1 | 27.5 | 35.0 | 50.6 |

TABLE 15 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 12—A—6—(a) | 630 | 76.4 | 25.2 | 2.5 | 61.3 | 27.7 |
| | 530 | 28.3 | 64.5 | 15.8 | 16.9 | 80.3 |
| | 500 | 11.4 | 45.1 | 36.7 | 15.0 | 81.8 |
| 12—A—6—(b) | 630/2 | 87.6 | 9.0 | 1.0 | 80.0 | 10.0 |
| | 530/2 | 33.4 | 62.5 | 5.9 | 24.5 | 68.4 |
| | 500/2 | 13.9 | 37.0 | 23.9 | 27.6 | 60.9 |
| 12—A—6—(c) | 600/2 | 93.7 | 2.2 | 0.3 | 94.8 | 2.5 |
| | 530 | 57.9 | 42.2 | 2.0 | 46.1 | 44.2 |
| | 500 | 28.7 | 49.7 | 3.0 | 38.1 | 52.7 |
| 12—A—6—(d) | 575/2 | 85.0 | 4.9 | 0.3 | 92.5 | 5.2 |
| | 530/2 | 52.7 | 28.7 | 0.6 | 63.3 | 29.3 |
| | 450/2 | 10.8 | 45.8 | 10.0 | 24.2 | 55.8 |
| 12—A—6—(e) | 575/2 | 93.0 | 0.6 | 0.0 | 90.0 | 0.6 |
| | 500/2 | 36.4 | 25.3 | 0.9 | 66.8 | 26.2 |
| | 400/2 | 2.4 | 51.8 | 17.5 | 35.8 | 69.3 |
| 12—A—6—(f) | 530/2 | 75.0 | 3.6 | 0.1 | 89.0 | 3.7 |
| | 400/2 | 4.5 | 39.6 | 11.9 | 56.5 | 51.5 |

TABLE 15 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 12—B—1—(a) | 630 | 80.0 | 27.8 | 0.1 | 42.0 | 27.9 |
| | 570 | 39.0 | 48.5 | 2.2 | 28.3 | 50.8 |
| | 450 | 2.7 | 31.0 | 17.0 | 31.0 | 48.0 |
| 12—B—1—(b) | 630 | 80.0 | 10.8 | 0.0 | 66.0 | 10.8 |
| | 570 | 16.0 | 27.7 | 6.3 | 58.1 | 34.0 |
| | 450 | 3.0 | 32.0 | 8.2 | 43.0 | 40.2 |
| 12—B—1—(c) | 630 | 91.4 | 2.8 | 0.0 | 83.0 | 2.8 |
| | 570 | 37.5 | 33.3 | 0.0 | 61.0 | 33.3 |
| | 450 | 2.7 | 32.3 | 4.0 | 41.9 | 36.3 |
| 12—B—1—(d) | 630 | 85.0 | 2.7 | 0.0 | 90.0 | 2.7 |
| | 570 | 26.5 | 55.5 | 0.0 | 38.0 | 55.5 |
| | 450 | 3.3 | 51.6 | 2.3 | 40.0 | 48.2 |
| 12—B—1—(e) | 630 | 34.7 | 15.4 | 1.0 | 83.6 | 16.4 |
| | 570 | 15.0 | 28.2 | 1.3 | 68.5 | 29.6 |
| | 450 | 2.6 | 47.5 | 11.0 | 19.0 | 58.5 |
| 13—B—1—(a) | 600 | 99.0 | 0.2 | 0.0 | 71.0 | 0.2 |
| | 570 | 88.0 | 12.6 | 0.3 | 51.0 | 12.9 |
| | 450 | 12.0 | 38.9 | 1.6 | 53.0 | 40.5 |

TABLE 15 (Cont'd)

| SAMPLE NO. | TEMPERATURE, °C./ CONTACT TIME SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 13—B—1—(b) | 570 | 92.0 | 8.7 | 0.0 | 54.9 | 8.7 |
| | 540 | 70.0 | 34.7 | 0.0 | 39.3 | 34.7 |
| | 450 | 7.0 | 68.9 | 2.6 | 24.0 | 71.5 |
| 13—B—1—(c) | 570 | 88.9 | 7.7 | 0.0 | 59.0 | 7.7 |
| | 450 | 4.0 | 70.6 | 4.3 | 19.7 | 74.9 |
| 13—B—1—(d) | 570 | 75.8 | 9.7 | 0.0 | 64.0 | 9.7 |
| | 450 | 2.7 | 52.7 | 4.0 | 27.6 | 56.7 |
| 13—B—1—(e) | 570 | 84.5 | 0.7 | 0.0 | 77.0 | 0.7 |
| | 450 | 3.2 | 40.5 | 0.0 | 43.0 | 40.5 |
| 14—A—1—(a) | 600 | 78.8 | 11.9 | 0.6 | 66.2 | 12.5 |
| | 570 | 50.8 | 49.7 | 3.1 | 28.4 | 52.8 |
| | 450 | 28.4 | 20.1 | 0.0 | 48.2 | 20.1 |
| 14—A—2—(a) | 630 | 90.0 | 8.5 | 0.0 | 89.6 | 8.5 |
| | 530 | 21.0 | 53.7 | 1.9 | 42.0 | 55.6 |
| | 500 | 17.4 | 66.3 | 0.2 | 30.3 | 66.5 |
| 14—A—3—(a) | 630 | 64.8 | 7.1 | 2.8 | 80.2 | 9.9 |
| | 575/2 | 17.0 | 28.5 | 16.2 | 40.8 | 44.7 |
| | 500 | 26.3 | 50.5 | 9.5 | 33.4 | 60.0 |

TABLE 15 (Cont'd)

| SAMPLE NO. | TEMPERATURE, °C./ CONTACT TIME, SECONDS[1] | CONVERSION % | SELECTIVITY, % | | | |
|---|---|---|---|---|---|---|
| | | | STILBENE | BIBENZYL | BENZENE | STILBENE + BIBENZYL |
| 14—A—4—(a) | 630 | 38.3 | 50.4 | 20.4 | 16.0 | 70.8 |
| | 575 | 20.2 | 50.7 | 35.0 | 8.6 | 85.7 |
| | 530 | 7.1 | 32.8 | 58.6 | 5.5 | 91.4 |
| | 500 | 2.8 | 21.0 | 69.7 | 6.8 | 90.7 |
| 14—A—5—(a) | 575 | 92.0 | 2.4 | 0.0 | 92.0 | 2.4 |
| | 500 | 34.2 | 38.8 | 0.8 | 69.4 | 39.6 |
| | 400 | 3.3 | 72.9 | 5.7 | 9.8 | 78.6 |

[1]A contact time of 4 seconds was employed unless otherwise noted.
[2]The catalytic mode of operation was employed, using an air/toluene mole ratio of 2/1 in the absence of added steam.

Example 16

*Procedure A* — A series of inorganic metal/oxygen compositions were prepared by intimately mixing the appropriate amount in grams of bismuth (III) oxide ($Bi_2O_3$) or bismuth (III) nitrate pentahydrate with at least one of each of an $M^4$ oxide (or hydroxide) or nitrate and an $M^3$ oxide (or hydroxide) or nitrate [or alternatively, mixing an appropriate amount in grams of a suitable bismuth-containing binary metal/oxygen composition with at least one $M^4$ oxide (or hydroxide) or nitrate, or an $M^3$ oxide (or hydroxide) or nitrate, depending on the element other than bismuth contained in the binary composition] in water and then heating to evaporate the water (and decompose the nitrates, when employed). The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 17 below. The parameters for such compositions, conveniently designated as 16—A—, are set forth in Table 16.

*Procedure B* — A series of inorganic metal/oxygen compositions supported on a calcium aluminate cement can be prepared according to the procedure described in Procedure A above except as follows. Following the final calcination, remove the calcined material from the oven, cool, crush, and mix with a calcium aluminate cement (Alcoa—CA—25) in an amount corresponding to 25% by weight of the calcined material. Slurry the solid mixture with water to form a thick paste and allow to air dry. Calcine the air-dried paste in air in an open casserole dish for 2 hours at 400°C. and then at a final temperature of 700°C. for an additional 2 hours. Remove the supported inorganic metal/oxygen composition from the oven, cool, crush in a mortar, and sieve to 14/30 mesh particles for evaluation in the toluene conversion reactor described in Example 17 below. Following these steps should result in compositions, conveniently designated as 16—B—, having properties beneficial for use in the process of this invention.

TABLE 16

| SAMPLE NO. | $BiM^4_bM^3_aO_x$ | STARTING MATERIALS GRAMS (MOLES) | | | CALCINATION CONDITIONS TEMPERATURE, °C./TIME HOURS | |
|---|---|---|---|---|---|---|
| | | Bi | $M^4$ | $M^3$ | INITIAL | FINAL |
| 16—A—1—(a) | $BiCa_{0.33}Zn_{0.33}O_x$ | $Bi_2O_3$ | CaO | ZnO | | |
| | | 139.8 (0.30) | 11.2 (0.20) | 16.3 (0.20) | 450/1 | 800/8 |
| 16—A—2—(a) | $BiCa_{0.5}KO_x$ | $Bi_2O_3$ | CaO | 85% KOH | | |
| | | 93.2 (0.20) | 11.2 (0.20) | 26.4 (0.40) | 450/1 | 800/8 |
| 16—A—3—(a) | $BiCa_{0.2}Ni_{0.33}O_x$ | $Bi_2O_3$ | CaO | NiO | | |
| | | 69.9 (0.15) | 3.4 (0.061) | 7.8 (0.10) | 450/1 | 800/8 |
| 16—A—4—(a) | $BiCa_{0.33}Ce_{0.033}O_x$ | $Bi_2O_3$ | CaO | $CeO_2$ | | |
| | | 139.8 (0.30) | 11.2 (0.20) | 3.4 (0.020) | 450/1 | 800/8 |
| 16—A—5—(a) | $BiCa_{0.33}Th_{0.33}O_x$ | $Bi_2O_3$ | CaO | $Th(NO_3)_4.4H_2O$ | | |
| | | 139.8 (0.30) | 11.2 (0.20) | 110.4 (0.20) | 450/1 | 800/8 |

Example 17

A. *Toluene Conversion Reactor* — A stainless steel tube 20.32 centimeters (8 inches) in length and 0.95 centimeter (0.375 inch) in internal diameter having a usable capacity of 11 milliliters was employed as a reactor for the toluene conversion reaction. The reactor was arranged vertically and equipped at the upper end with reactant inlet means having calibrated flow controllers and vaporizers, and at the lower end with reaction effluent outlet means for collecting the reaction effluent or, alternatively, for direct introduction thereof via a gas sampling valve into a gas-liquid chromatograph for analysis. The outlet means was also equipped with means for introducing an inert gas diluent — nitrogen or helium, for example — into the reaction effluent for analysis purposes. A radiant furnace was used to maintain a constant temperature during the reaction period. The temperature was measured with a thermocouple in a temperature well located on the lower outside wall of the reactor.

B. *Toluene Conversion* — The reaction was conducted in the stoichiometric mode of operation unless otherwise noted. The reactor was charged with approximately 11 milliliters of the inorganic metal/oxygen composition prepared as described in Example 16 above. Glass wool plugs were used as supports for the composition. The charged reactor was placed in a radiant furnace and heated to maintain a constant temperature throughout the reaction period. Steam and toluene in a 2:1 mole ratio were fed to the reactor at a pressure of $1.013 \times 10^5$ pascal (1 atmosphere) at a rate sufficient to provide a reactor residence (contact) time of 4 seconds (unless otherwise noted) for the toluene (assuming a 50% void space in the reactor). After the reaction had proceeded for 1 minute, the reaction effluent, diluted with helium, was analyzed by gas-liquid chromatography. The results are tabulated in Table 17.

TABLE 17

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 16—A—1—(a) | 630/2 | 41.6 | 52.6 | 15.2 | 18.9 | 67.8 |
| | 575/2 | 15.3 | 37.5 | 39.9 | 16.0 | 77.4 |
| | 500/2 | 3.6 | 18.5 | 64.4 | 11.5 | 82.9 |
| 16—A—2—(a) | 595/2 | 7.5 | 43.0 | 28.2 | 31.8 | 71.2 |
| | 575/2 | 9.1 | 69.3 | 17.7 | 15.1 | 87.0 |
| | 475 | 27.5 | 77.7 | 4.5 | 15.6 | 82.2 |
| 16—A—3—(a) | 630 | 78.9 | 34.9 | 1.4 | 47.1 | 36.3 |
| | 575 | 62.3 | 62.5 | 1.3 | 28.3 | 63.8 |
| | 500 | 15.1 | 72.0 | 12.2 | 11.5 | 84.2 |
| 16—A—4—(a) | 630/2 | 97.5 | 4.0 | 0.3 | 58.0 | 4.3 |
| | 530 | 43.1 | 62.3 | 5.4 | 15.0 | 67.7 |
| | 450 | 6.0 | 32.9 | 32.0 | 7.3 | 64.9 |
| 16—A—5—(a) | 600 | 93.0 | 2.9 | 0.0 | 97.6 | 2.9 |
| | 530 | 60.7 | 50.0 | 0.5 | 47.2 | 50.5 |
| | 500/2 | 20.9 | 55.8 | 4.0 | 36.6 | 59.8 |

[1]A contact time of 4 seconds was employed unless otherwise noted.

Example 18

A series of inorganic metal/oxygen compositions were prepared by intimately mixing the appropriate amount in grams of lead (II) oxide (PbO), bismuth (III) oxide ($Bi_2O_3$) (as $M^2$), and at least one $M^1$ oxide (or hydroxide) in water, filtering to remove excess water or, alternatively, heating to evaporate the water, and then drying. The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 20 below. The parameters for such compositions, conveniently designated as 18—A—, are set forth in Table 18.

TABLE 18

| | | STARTING MATERIALS GRAMS (MOLES) | | | CALCINATION CONDITIONS TEMPERATURE, °C./TIME, HOURS | |
|---|---|---|---|---|---|---|
| SAMPLE NO. | $PbM^2_bM^1_aO_x$ | Pb | $M^2$ | $M^1$ | INITIAL | FINAL |
| 18—A—1—(a) | $PbBi_{0.33}Zr_{0.33}O_x$ | PbO | $Bi_2O_3$ | $ZrO_2$ | | |
| | | 80.0 (0.36) | 28.0 (0.060) | 15.0 (0.12) | 400/1 | 700/8 |
| 18—A—2—(a) | $PbBi_{0.33}As_{0.16}O_x$ | PbO | $Bi_2O_3$ | $As_2O_3$ | | |
| | | 100.0 (0.45) | 35.0 (0.075) | 7.4 (0.037) | 400/1 | 800/4 |
| 18—A—3—(a) | $PbBi_{0.33}Zn_{0.33}O_x$ | PbO | $Bi_2O_3$ | ZnO | | |
| | | 100.0 (0.45) | 35.0 (0.075) | 12.2 (0.15) | 400/1 | 750/5 |
| 18—A—4—(a) | $PbBiHo_{0.25}O_x$ | PbO | $Bi_2O_3$ | $Ho_2O_3$ | | |
| | | 60.0 (0.27) | 63.0 (0.14) | 13.0 (0.034) | 400/1 | 800/8 |
| 18—A—5—(a) | $PbBi_3Y_4O_x$ | PbO | $Bi_2O_3$ | $Y_2O_3$ | | |
| | | 17.3 (0.078) | 54.5 (0.12) | 35.0 (0.16) | 400/1 | 700/5 |
| 18—A—6—(a) | $PbBiSr_2O_x$ | PbO | $Bi_2O_3$ | SrO | | |
| | | 67.0 (0.30) | 69.9 (0.15) | 62.2 (0.60) | 450/1 | 800/8 |
| 18—A—7—(a) | $PbBiGe_{0.25}O_x$ | PbO | $Bi_2O_3$ | $GeO_2$ | | |
| | | 89.3 (0.40) | 93.2 (0.20) | 10.5 (0.10) | 450/1 | 800/8 |
| 18—A—8—(a) | $PbBi_3In_{0.3}O_x$ | PbO | $Bi_2O_3$ | $In_2O_3$ | | |
| | | 44.6 (0.20) | 139.8 (0.30) | 8.3 (0.030) | 450/1 | 800/8 |
| 18—A—9—(a) | $PbBi_3NiO_x$ | PbO | $Bi_2O_3$ | NiO | | |
| | | 44.6 (0.20) | 139.8 (0.30) | 14.9 (0.20) | 450/1 | 800/8 |

Example 19

*Procedure A* — The appropriate amounts in grams of lead (II) nitrate [$Pb(NO_3)_2$], bismuth (III) nitrate pentahydrate [$Bi(NO_3)_3.5H_2O$] (as $M^2$), and at least one $M^1$ nitrate were dissolved in approximately 100 milliliters of concentrated nitric acid ($HNO_3$) and heated to evaporate and decompose the nitric acid and nitrates. The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 20 below. The parameters for one such composition, conveniently designated as 19—A—, are set forth in Table 19.

*Procedure B* — The procedure described in Procedure A above was employed except that following the final calcination, the calcined material was removed from the oven, cooled, crushed, and mixed with a calcium aluminate cement (Alcoa—CA—25) in an amount corresponding to 25% by weight of the dry weight of the calcined material. The resulting solid mixture was slurried with water to form a thick paste and allowed to air dry. The air-dried paste was calcined in air in an open casserole dish for 2 hours at 400°C. and then at a final temperature of 700°C. for an additional 2 hours. The supported inorganic metal/oxygen composition was removed from the oven, cooled, crushed in a mortar, and sieved to 14/30 mesh particles for evaluation in the toluene conversion reactor described in Example 20 below. The parameters for one such composition, conveniently designated as 19—B, are set forth in Table 19.

TABLE 19

| | | STARTING MATERIALS GRAMS (MOLES) | | | CALCINATION CONDITIONS TEMPERATURE, °C./TIME, HOURS | |
|---|---|---|---|---|---|---|
| SAMPLE NO. | $PbM^2_bM^1_aO_x$ | Pb | $M^2$ | $M^1$ | INITIAL | FINAL |
| 19—A—1—(a) | $PbBi_6Th_3O_x$ | $Pb(NO_3)_2$ | $Bi(NO_3)_3.5H_2O$ | $Th(NO_3)_4.H_2O$ | | |
| | | 16.6 (0.050) | 145.5 (0.30) | 82.2 (0.15) | 450/1 | 800/8 |
| 19—B—1—(a) | $PbBiAg_{0.15}O_x$ | $Pb(NO_3)_2$ | $Bi(NO_3)_3.5H_2O$ | $AgNO_3$ | | |
| | | 60.0 (0.19) | 88.0 (0.18) | 5.0 (0.029) | 450/1 | 800/10 |

Example 20

A. *Toluene Conversion Reactor* — A stainless steel tube 20.32 centimeters (8 inches) in length and 0.95 centimeter (0.375 inch) in internal diameter having a usable capacity of 11 milliliters was employed as a reactor for the toluene conversion reaction. The reactor was arranged vertically and equipped at the upper end with reactant inlet means having calibrated flow controllers and vaporizers, and at the lower end with reaction effluent outlet means for collecting the reaction effluent or, alternatively, for direct introduction thereof via a gas sampling valve into a gas-liquid chromatograph for analysis. The outlet means was also equipped with means for introducing an inert gas diluent — nitrogen or helium, for example — into the reaction effluent for analysis purposes. A radiant furnace was used to maintain a constant temperature during the reaction period. The temperature was measured with a thermocouple in a temperature well located on the lower outside wall of the reactor.

B. *Toluene Conversion* — The reaction was conducted in the stoichiometric mode of operation unless otherwise noted. The reactor was charged with approximately 11 milliliters of the inorganic metal/oxygen composition prepared as described in Examples 18 and 19 above. Glass wool plugs were used as supports for the composition. The charged reactor was placed in a radiant furnace and heated to maintain a constant temperature throughout the reaction period. Steam and toluene in a 2:1 mole ratio were fed to the reactor at a pressure of $1.013 \times 10^5$ pascal (1 atmosphere) at a rate sufficient to provide a reactor residence (contact) time of 4 seconds (unless otherwise noted) for the toluene (assuming a 50% void space in the reactor). After the reaction had proceeded for 1 minute, the reaction effluent, diluted with helium, was analyzed by gas-liquid chromatography. The results are tabulated in Table 20.

TABLE 20

| SAMPLE NO. | TEMPERATURE, °C./ CONTACT TIME SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 18—A—1—(a) | 630 | 19.9 | 18.0 | 34.4 | 14.5 | 52.4 |
| | 540 | 9.2 | 36.4 | 41.9 | 10.3 | 78.3 |
| | 450 | 0.4 | 0.0 | 16.0 | 18.5 | 16.0 |
| 18—A—2—(a) | 630 | 35.7 | 39.4 | 16.5 | 21.3 | 55.9 |
| | 540 | 5.6 | 22.4 | 46.0 | 15.0 | 68.4 |
| | 450 | 0.7 | 0.0 | 17.1 | 11.4 | 17.1 |
| 18—A—3—(a) | 630 | 17.4 | 16.6 | 35.1 | 12.3 | 51.7 |
| | 570 | 5.3 | 15.0 | 58.9 | 10.1 | 73.9 |
| | 450 | 0.6 | 0.0 | 16.1 | 25.5 | 16.1 |
| 18—A—4—(a) | 630 | 35.8 | 22.0 | 18.5 | 15.4 | 40.5 |
| | 570 | 16.1 | 19.6 | 31.2 | 4.6 | 50.8 |
| | 450 | 0.6 | 0.0 | 15.1 | 40.1 | 15.1 |
| 18—A—5—(a) | 630 | 87.6 | 43.4 | 0.0 | 40.9 | 43.4 |
| | 570 | 41.7 | 65.3 | 5.4 | 19.2 | 70.7 |
| | 450 | 7.1 | 5.2 | 7.5 | 4.9 | 12.7 |
| 18—A—6—(a) | 630/2 | 95.4 | 35.6 | 0.0 | 30.3 | 35.6 |
| | 575/2 | 68.1 | 69.0 | 9.6 | 5.3 | 78.6 |
| | 500/2 | 3.8 | 20.8 | 57.4 | 7.4 | 78.2 |

TABLE 20 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENYL |
| 18—A—7—(a) | 575/2 | 13.9 | 31.3 | 48.1 | 6.8 | 79.4 |
| | 500/2 | 3.4 | 12.6 | 60.9 | 2.0 | 73.5 |
| 18—A—8—(a) | 630 | 27.2 | 30.4 | 27.2 | 15.5 | 57.6 |
| | 575 | 23.9 | 49.7 | 24.9 | 9.1 | 74.6 |
| | 500 | 4.3 | 15.3 | 55.7 | 4.3 | 71.0 |
| 18—A—9—(a) | 630/2 | 40.7 | 26.1 | 14.6 | 17.8 | 40.7 |
| | 575/2 | 28.6 | 52.2 | 21.1 | 8.6 | 73.3 |
| | 500/2 | 4.5 | 21.1 | 55.3 | 9.2 | 76.4 |
| 19—A—1—(a) | 630 | 97.0 | 7.0 | 0.0 | 71.0 | 7.0 |
| | 530 | 48.3 | 71.7 | 2.3 | 11.8 | 74.0 |
| | 500 | 31.8 | 57.9 | 8.0 | 8.0 | 65.9 |
| 19—B—1—(a) | 630 | 43.1 | 27.9 | 14.4 | 21.1 | 42.3 |
| | 575 | 22.8 | 38.3 | 30.3 | 10.8 | 68.6 |
| | 450 | 2.1 | 6.3 | 33.9 | 7.4 | 40.2 |

[1]A contact time of 4 seconds was employed unless otherwise noted.

Example 21

*Procedure A* — A series of inorganic metal/oxygen compositions were prepared by intimately mixing the appropriate amount of lead (II) oxide (PbO), lead (II) carbonate ($PbCO_3$), or lead (II) nitrate [$Pb(NO_3)_2$] with at least one of each of an $M_2$ carbonate, nitrate, or oxide and an $M^1$ carbonate, nitrate, or oxide (or, alternatively, mixed an appropriate amount of a suitable lead-containing binary metal/oxygen composition with at least one $M^2$ carbonate, nitrate, or oxide or one $M^1$ carbonate, nitrate, or oxide, depending on the element other than lead contained in the binary composition) in water and heating to evaporate the water (and decompose the carbonates and/or nitrates, when employed). The resulting solid was placed in an open casserole dish and calcined in air for an initial period at an initial temperature and then at a final temperature for an additional period. The calcined material was removed from the oven, cooled, crushed in a mortar, and sieved to 14/30 mesh particles for evaluation in the toluene conversion reactor described in Example 22 below. The parameters for such compositions, conveniently designated as 21—A—, are set forth in Table 21.

*Procedure B* — A series of inorganic metal/oxygen compositions supported on a calcium aluminate cement can be prepared according to the procedure described in Procedure A above except as follows. Following the final calcination, remove the calcined material from the oven, cool, crush, and mix with a calcium aluminate cement (Alcoa—CA—25) in an amount corresponding to 25% by weight of the calcined material. Slurry the solid mixture with water to form a thick paste and allow to air dry. Calcine the air-dried paste in air in an open casserole dish for 2 hours at 400°C. and then at a final temperature of 700°C. for an additional 2 hours. Remove the supported inorganic metal/oxygen composition from the oven, cool, crush in a mortar, and sieve to 14/30 mesh particles for evaluation in the toluene conversion reactor described in Example 22 below. Following these steps should result in compositions having properties beneficial for use in the process of this invention.

TABLE 21

| SAMPLE NO. | Pb $M^2_bM^1_aO_x$ | STARTING MATERIALS GRAMS (MOLES) Pb | $M^2$ | $M^1$ | CALCINATION CONDITIONS TEMPERATURE, °C./TIME, HOURS INITIAL | FINAL |
|---|---|---|---|---|---|---|
| 21—A—1—(a) | $PbSO_{0.33}As_{0.33}O_x$ | PbO | $Sb_2O_3$ | $As_2O_3$ | | |
| | | 133.1 (0.60) | 29.2 (0.10) | 19.8 (0.10) | 450/1 | 800/8 |
| 21—A—2—(a) | $PbSO_{0.33}Ce_{0.017}O_x$ | PbO | $Sb_2O_3$ | $CeO_2$ | | |
| | | 67.0 (0.30) | 15.4 (0.053) | 0.9 (0.0052) | 450/1 | 800/8 |
| 21—A—3—(a) | $PbSb_{0.25}Co_{0.25}O_x$ | $Pb_4SbO_x$[1] | — | $CoCO_3$ | | |
| | | 116.0 (0.056) | — | 6.7 (0.056) | 450/1 | 800/8 |
| 21—A—4—(a) | $PbSb_{0.25}Ga_{0.25}O_x$ | PbO | $Sb_2O_3$ | $Ga_2O_3$ | | |
| | | 95.3 (0.43) | 15.6 (0.054) | 10.0 (0.053) | 400/1 | 830/4 |
| 21—A—5—(a) | $PbSb_{0.48}La_{0.48}O_x$ | PbO | $Sb_2O_3$ | $La_2O_3$ | | |
| | | 116.6 (0.50) | 36.4 (0.12) | 40.7 (0.12) | 400/1 | 830/4 |
| 21—A—6—(a) | $PbSb_{0.25}Ag_{0.25}O_x$ | PbO | $Sb_2O_3$ | $AgNO_3$ | | |
| | | 100.0 (0.45) | 16.3 (0.056) | 19.0 (0.11) | 450/1 | 800/6 |
| 21—A—7—(a) | $PbSb_{0.33}Sr_{0.33}O_x$ | PbO | $Sb_2O_3$ | SrO | | |
| | | 133.1 (0.60) | 29.2 (0.10) | 20.7 (0.20) | 450/1 | 800/8 |
| 21—A—8—(a) | $PbSb_{0.5}Zn_{0.5}O_x$ | PbO | $Sb_2O_3$ | ZnO | | |
| | | 178.5 (0.80) | 58.3 (0.20) | 32.5 (0.40) | 450/1 | 800/8 |

[1] The lead antimonate binary composition, having a (calculated) gram molecular weight of 2077.0 grams, was prepared as follows:

Lead (III) oxide (PbO; 178.4 grams, 0.080 mole) and antimony (III) oxide ($Sb_2O_3$; 29.2 grams, 0.10 mole) were intimately mixed in water and then heated to evaporate the water. The resulting solid was placed in an open casserole dish and calcined in air for 2 hours at 400°C. and then at 800°C. for an additional 8 hours. The calcined material was removed from the oven, cooled, crushed in a mortar, and ground to a fine powder.

Example 22

A. *Toluene Conversion Reactor* — A stainless steel tube 20.32 centimeters (8 inches) in length and 0.95 centimeter (0.375 inch) in internal diameter having a usable capacity of 11 milliliters was employed as a reactor for the toluene conversion reaction. The reactor was arranged vertically and equipped at the upper end with reactant inlet means having calibrated flow controllers and vaporizers, and at the lower end with reaction effluent outlet means for collecting the reaction effluent or, alternatively, for direct introduction thereof via a gas sampling valve into a gas-liquid chromatograph for analysis. The outlet means was also equipped with means for introducing an inert gas diluent — nitrogen or helium, for example — into the reaction effluent for analysis purposes. A radiant furnace was used to maintain a constant temperature during the reaction period. The temperature was measured with a thermocouple in a temperature well located on the lower outside wall of the reactor.

B. *Toluene Conversion* — The reaction was conducted in the stoichiometric mode of operation unless otherwise noted. The reactor was charged with approximately 11 milliliters of the inorganic metal/oxygen composition prepared as described in Example 21 above. Glass wool plugs were used as supports for the composition. The charged reactor was placed in a radiant furnace and heated to maintain a constant temperature throughout the reaction period. Steam and toluene in a 2:1 mole ratio were fed to the reactor at a pressure of $1.013 \times 10^5$ pascal (1 atmosphere) at a rate sufficient to provide a reactor residence (contact) time of 4 seconds (unless otherwise noted) for the toluene (assuming a 50% void space in the reactor). After the reaction had proceeded for 1 minute, the reaction effluent, diluted with helium, was analyzed by gas-liquid chromatography. The results are tabulated in Table 22.

TABLE 22

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 21—A—1—(a) | 630/2 | 14.7 | 13.2 | 31.1 | 21.7 | 44.3 |
| | 575/2 | 12.2 | 17.4 | 32.9 | 24.0 | 50.3 |
| | 500/2 | 2.3 | 9.1 | 48.2 | 24.7 | 57.3 |
| 21—A—2—(a) | 630/2 | 54.5 | 37.4 | 8.4 | 22.0 | 45.8 |
| | 575/2 | 13.5 | 29.2 | 34.4 | 21.2 | 63.6 |
| | 500/2 | 5.3 | 24.2 | 46.4 | 16.8 | 70.6 |
| 21—A—3—(a) | 630 | 44.4 | 42.0 | 11.7 | 24.0 | 53.7 |
| | 540 | 15.6 | 32.5 | 27.0 | 20.7 | 59.5 |
| | 450 | 1.6 | 3.2 | 16.1 | 15.5 | 19.3 |
| 21—A—4—(a) | 630 | 17.7 | 26.6 | 24.8 | 27.6 | 51.4 |
| | 540 | 6.4 | 30.1 | 33.0 | 25.4 | 63.1 |
| | 450 | 0.7 | 0.0 | 11.6 | 55.7 | 11.6 |
| 21—A—5—(a) | 630/2 | 94.0 | 17.0 | 0.2 | 73.0 | 17.2 |
| | 575/2 | 65.8 | 48.1 | 0.8 | 46.8 | 48.9 |
| | 500/2 | 29.9 | 50.7 | 2.1 | 36.9 | 52.8 |
| 21—A—6—(a) | 630 | 50.9 | 13.2 | 6.3 | 35.0 | 19.5 |
| | 540 | 16.2 | 30.6 | 29.3 | 13.2 | 59.9 |
| | 450 | 2.1 | 8.8 | 41.2 | 17.9 | 50.0 |

TABLE 22 (Cont'd)

| SAMPLE NO. | TEMPERATURE, °C./ CONTACT TIME, SECONDS[1] | CONVERSION, % | SELECTIVITY, % | | | |
|---|---|---|---|---|---|---|
| | | | STILBENE | BIBENZYL | BENZENE | STILBENE + BIBENZYL |
| 21—A—7—(a) | 630/2 | 83.4 | 9.5 | 0.0 | 83.9 | 9.5 |
| | 575/2 | 47.7 | 34.9 | 0.0 | 53.8 | 34.9 |
| | 500/2 | 13.5 | 52.3 | 0.0 | 38.8 | 52.3 |
| 21—A—8—(a) | 630/2 | 35.9 | 36.5 | 6.8 | 30.6 | 43.3 |
| | 575/2 | 10.3 | 31.6 | 27.7 | 20.8 | 59.3 |
| | 500/2 | 5.4 | 41.7 | 29.3 | 12.9 | 71.0 |

[1] A contact time of 4 seconds was employed unless otherwise noted.

Thus, it is apparent that there has been provided, in accordance with the present invention, a process that fully satisfies the objects and advantages set forth hereinabove. While the invention has been described with respect to various specific examples and embodiments thereof, it is understood that the invention is not limited thereto.

## Claims

1. A process for dehydrocupling toluene which comprises contacting the toluene in the vapor phase at a temperature between 450°C. and 650°C. with an inorganic metal/oxygen composition characterized by an empirical formula selected from:

(a) $$Pb\ M^1_a\ M^2_b\ O_x$$

where $M^1$ is at least one element selected from silver, zinc, gallium, indium, thallium, germanium, phosphorus, arsenic, thorium, the lanthanides, Groupes 1a, 2a, 3b, 4b, and 8 of the Periodic Table of the Elements and mixtures thereof, and $M^2$ is an element selected from bismuth and antimony, and wherein a is 0.01 to 10, b is 0 to 10, and x is a number taken to satisfy the average valences of lead, $M^1$ and $M^2$ in the oxidation states in which they exist in the composition, with the proviso that when $M^2$ is bismuth, $M^1$ cannot be gallium or thallium; and when $M^2$ is antimony, $M^1$ cannot be indium, thallium, germanium, phosphorus, or thorium; and

(b) $$Bi\ M^3_a\ M^4_b\ O_x$$

where $M^3$ is at least one element selected from zinc, germanium, thorium, the lanthanides, Groups 1a, 3B, 4b, and 8 of the Periodic Table of the Elements and mixtures thereof, and $M^4$ is at least one element selected from indium, silver, Group 2a of the Period Table of the Elements, and mixtures thereof, and wherein a is 0.01 to 10, b is 0 to 10, and x is a number taken to satisfy the average valences of bismuth, $M^3$, and $M^4$ in the oxidation state in which they exist in the composition to yield the toluene dehydrocoupled toluene product.

2. The process of Claim 1 characterized in that $M^1$ is selected from silver, zinc, lithium, sodium, potassium, rubidium, cesium, magnesium, calcium, strontium, barium, indium, arsenic, the lanthanides and Groupes 3b, 4b, and 8 of the Periodic Table of the Elements, and mixture thereof.

3. The process of Claim 2 characterized in that $M^1$ is selected from silver, zinc, potassium, zirconium, barium, calcium, strontium, nickel, platinum and cerium, and mixtures thereof.

4. The process of Claim 1 characterized in that $M^2$ is bismuth.

5. The process of Claim 1 characterized in that $M^3$ is selected from zinc, the lanthanides and Groups 3b, 4b, and 8 of the Periodic Table of the Elements, and mixtures thereof.

6. The process of Claim 5 characterized in that $M^3$ is selected from zinc, cerium, zirconium, cobalt, nickel, and platinum, and mixtures thereof.

7. The process of Claim 1 characterized in that $M^4$ is selected from indium, silver, magnesium, calcium, strontinum and barium, and mixtures thereof.

8. The process of Claim 7 characterized in that $M^4$ is selected from silver, calcium, strontinum and barium, and mixtures thereof.

9. The process of Claim 1 characterized in that steam is introduced with the toluene in an amount sufficient to provide a steam-to-toluene mole ratio between 0.1 and 10.

10. The process of Claim 1 characterized in that a and b in the empirical formulas are 0.5 to 5.

11. The process of Claim 1 characterized in that the contacting between the toluene and the inorganic metal/oxygen composition is effected for a period between 1 second and 12 seconds.

12. The process of Claim 1 characterized in that the temperature is between 500°C. and 600°C.

13. The process of Claim 1 characterized in that the dehydrocoupling reaction is conducted in a stoichiometric mode of operation in the absence of added free oxygen.

14. The process of Claim 1 characterized in that a reactant selected from the group consisting of oxygen and an oxygen-containing gas is introduced with the toluene.

15. The process of Claim 14 characterized in that the oxygen and oxygen-containing gas is introduced in an amount sufficient to conduct the dehydrocoupling reaction in a catalytic mode of operation.

16. The process of Claim 15 characterized in that the oxygen and oxygen-containing gas is introduced in an amount sufficient to provide a toluene-to-oxygen mole ratio between about 1 and 8.

17. The process of Claim 14 characterized in that the oxygen and oxygen-containing gas is introduced in an amount sufficient to conduct the dehydrocoupling reaction in a combined catalytic/stoichiometric mode of operation.

18. The process of Claim 1 characterized in that the inorganic metal/oxygen composition is admixed with a support material.

19. The process of Claim 18 characterized in that the support material is a metal aluminate.

20. The process of Claim 19 characterized in that the metal aluminate is calcium aluminate.

21. The process of Claim 1 characterized in that the dehydrocoupling reaction is conducted at a toluene conversion level of about 20 to about 55 percent.

22. The process of Claims 1 through 16 characterized in that the dehydrocoupled toluene product is stilbene.

**Patentansprüche**

1. Verfahren zur dehydrierenden Kupplung von Toluol durch Inkontaktbringen des Toluols in der Dampfphase bei einer Temperatur zwischen 450°C und 650°C mit einer anorganischen Metall/Sauerstoff-Zubereitung, gekennzeichnet durch eine Summenformel, die ausgewählt ist aus

(a) $$Pb\, M^1_a\, M^2_b\, O_x,$$

worin $M^1$ mindestens ein Element ausgewählt aus Silber, Zink, Gallium, Indium, Thallium, Germanium, Phosphor, Arsen, Thorium, den Lanthaniden, den Gruppen 1a, 2a, 3b, 4b und 8 des Periodensystems der Elemente und Mischungen davon und $M^2$ eine Element ausgewählt aus Wismut und Antimon darstellen und worin a 0,01 bis 10, b 0 bis 10 und x eine Zahl mit einem solchen Wert darstellen, daß die durchschnittlichen Wertigkeiten von Blei, $M^1$ und $M^2$ in den Oxidationszuständen, in denen sie in der Zubereitung vorliegen, befriedigt sind, mit der Maßgabe, daß, wenn $M^2$ Wismut ist, $M^1$ nicht Gallium oder Thallium bedeutet; und wenn $M^2$ Antimon ist, $M^1$ nicht Indium, Thallium, Germanium Phosphor oder Thorium bedeutet; und

(b) $$Bi\, M^3_a\, M^4_b\, O_x,$$

worin $M^3$ mindestens ein Element ausgewählt aus Zink, Germanium, Thorium, den Lanthaniden, den Gruppen 1a, 3b, 4b und 8 des Periodensystems der Elemente und Mischungen davon und $M^4$ mindestens ein Element ausgewählt aus Indium, Silber, der Gruppe 2a des Periodensystems der Elemente und Mischungen davon darstellen und worin a 0,01 bis 10, b 0 bis 10 und x eine Zahl mit einem solchen Wert bedeuten, daß die durchschnittlichen Wertigkeiten von Wismut, $M^3$ und $M^4$ in dem Oxidationszustand, in dem sie in der Zubereitung vorliegen, befriedigt ist, zur Bildung des durch dehydrierende Kupplung von Toluol gebildeten Produkts.

2. Verfahren nach Anspruch 1, dadurch gekennzeichnet, daß $M^1$ ausgewählt ist aus Silber, Zink, Lithium, Natrium, Kalium, Rubidium, Cäsium, Magnesium, Calcium, Strontium, Barium, Indium, Arsen, den Lanthaniden und den Gruppen 3b, 4b und 8 des Periodensystems der Elemente und Mischungen davon.

3. Verfahren nach Anspruch 2, dadurch gekennzeichnet, daß $M^1$ ausgewählt ist aus Silber, Zink, Kalium, Zirkonium, Barium, Calcium, Strontium, Nickel, Platin und Cer und Mischungen davon.

4. Verfahren nach Anspruch 1, dadurch gekennzeichnet, daß $M^2$ Wismut bedeutet.

5. Verfahren nach Anspruch 1, dadurch gekennzeichnet, daß $M^3$ aus Zink, den Lanthaniden und den Gruppen 3b, 4b und 8 des Periodensystems der Elemente und Mischungen davon ausgewählt ist.

6. Verfahren nach Anspruch 5, dadurch gekennzeichnet, daß $M^3$ ausgewählt ist aus Zink, Cer, Zirkonium, Kobalt, Nickel und Platin und Mischungen davon.

7. Verfahren nach Anspruch 1, dadurch gekennzeichnet, daß $M^4$ ausgewählt ist aus Indium, Silber, Magnesium, Calcium, Strontium und Barium und Mischungen davon.

8. Verfahren nach Anspruch 7, dadurch gekennzeichnet, daß $M^4$ ausgewählt ist aus Silber, Calcium, Strontium und Barium und Mischungen davon.

9. Verfahren nach Anspruch 1, dadurch gekennzeichnet, daß Wasserdampf mit Toluol in einer Menge eingeführt wird, die dazu ausreicht, ein Wasserdampf/Toluol-Molverhältnis zwischen 0,1 und 10 zu ergeben.

10. Verfahren nach Anspruch 1, dadurch gekennzeichnet, daß a und b in den Summenformeln 0,5 bis 5 betragen.

11. Verfahren nach Anspruch 1, dadurch gekennzeichnet, daß der Kontakt des Toluols mit der anorganischen Metall/Sauerstoff-Zubereitung während einer Zeitdauer zwischen 1 Sekunde und 12 Sekunden bewirkt wird.

12. Verfahren nach Anspruch 1, dadurch gekennzeichnet, daß die Temperatur zwischen 500°C und 600°C liegt.

13. Verfahren nach Anspruch 1, dadurch gekennzeichnet, daß die dehydrierende Kupplungsreaktion in Abwesentheit von zugesetztem freiem Sauerstoff unter stöchiometrischen Bedingungen durchgeführt wird.

14. Verfahren nach Anspruch 1, dadurch gekennzeichnet, daß zusammen mit dem Toluol ein Reaktionsteilnehmer eingeführt wird, der aus der Gruppe ausgewählt ist, die Sauerstoff und ein sauerstoffhaltiges Gas umfaßt.

15. Verfahren nach Anspruch 14, dadurch gekennzeichnet, daß der Sauerstoff und das sauerstoffhaltige Gas in einer Menge eingeführt werden, die dazu ausreicht, die dehydrierende Kupplungsreaktion entsprechend einer katalytischen Umsetzung zu bewirken.

16. Verfahren nach Anspruch 15, dadurch gekennzeichnet, daß der Sauerstoff und das Sauerstoff enthaltende Gas in einer Menge eingeführt werden, die dazu ausreicht, ein Toluol/Sauerstoff-Molverhältnis zwischen etwa 1 und 8 zu ergeben.

17. Verfahren nach Anspruch 14, dadurch gekennzeichnet, daß der Sauerstoff und das sauerstoffhaltige Gas in einer Menge eingeführt werden, die dazu ausreicht, die dehydrierende Kupplungsreaktion entsprechend einer kombinierten katalytischen/stöchiometrischen Umsetzung ablaufen zu lassen.

18. Verfahren nach Anspruch 1, dadurch gekennzeichnet, daß die anorganische Metall/Sauerstoff-Zubereitung mit einem Trägermaterial vermischt wird.

19. Verfahren nach Anspruch 18, dadurch gekennzeichnet, daß das Trägermaterial ein Metallaluminat ist.

20. Verfahren nach Anspruch 19, dadurch gekennzeichnet, daß das Metallaluminat Calciumaluminat ist.

21. Verfahren nach Anspruch 1, dadurch gekennzeichnet, daß die dehydrierende Kupplungsreaktion bei einer Toluolumwandlung von etwa 20 bis etwa 55% durchgeführt wird.

22. Verfahren nach den Ansprüchen 1 bis 16 dadurch gekennzeichnet, daß das durch dehydrierende Kupplung von Toluol gebildete Produkt Stilben ist.

**Revendications**

1. Procédé pour le déhydrocouplage de toluène qui consiste à mettre en contact le toluène en phase vapeur à une température comprise entre 450°C et 650°C avec une composition minérale métal/oxygène, caractérisé par une formule empirique choisie parmi:

(a)

$$Pb\, M^1_a\, M^2_b\, O_x$$

où $M^1$ est au moins un élément choisi parmi l'argent, le zinc, le gallium, l'indium, le thallium, le germanium, le phosphore, l'arsenic, le thorium, les lantanides, les groupes 1a, 2a, 3b, 4b et 8 du tableau de classification périodique des élements et leurs mélanges, et $M^2$ est un élément choisi parmi le bismuth et l'antimoine, et où a vaut 0,01 à 10, b vaut 0 à 10 et x est un nombre choisi pour satisfaire aux valences moyennes du plomb, de $M^1$ et de $M^2$ dans les états d'oxydation dans lesquels ils existent dans la composition, en prévoyant que lorsque $M^2$ est le bismuth, $M^1$ ne peut pas être le gallium ou le thallium, et lorsque $M^2$ est l'antimoine, $M^1$ ne peut pas être l'indium; le thallium, le germanium, le phosphore ou le thorium; et

(b)

$$Bi\, M^3_a\, M^4_b\, O_x$$

où $M^3$ est au moins un élément choisi parmi le zinc, le germanium, le thorium, les lanthanides, les groupes 1a, 3b, 4b, et 8 du tableau de classification périodique des éléments et leurs mélanges, et $M^4$ est au moins un élément choisi parmi l'indium, l'argent, le groupe 2a du tableau de classification périodique des éléments et leurs mélanges, et où a vaut 0,01 à 10, b vaut 0 à 10, et: est un nombre choisi pour satisfaire aux valences moyennes du bismuth, de $M^3$ et $M^4$ dans l'état d'oxydation dans lequel ils existent dans la composition pour fournir le produit déhydrocouplé du toluène.

2. Procédé selon la revendication 1, caractérisé en ce que $M^1$ est choisi parmi l'argent, le zinc, le lithium, le sodium, le potassium, le rubidium, le césium, le magnésium, le calcium, le strontium, le baryum, l'indium, l'arsenic, les lanthanides et les groupes 3b, 4b et 8 du tableau de classification périodique des éléments, et leurs mélanges.

3. Procédé selon la revendication 2, caractérisé en ce que $M^1$ est choisi parmi l'argent, le zinc, le potassium, le zirconium, le baryum, le calcium, le strontium, le nickel, le platine et le cérium, et leurs mélanges.

4. Procédé selon la revendication 1, caractérisé en ce que $M^2$ est le bismuth.

5. Procédé selon la revendication 1, caractérisé en ce que $M^3$ est choisi parmi le zinc, les lanthanides, et les groupes 3b, 4b et 8 du tableau de classification périodique des éléments, et leurs mélanges.

6. Procédé selon la revendication 5, caractérisé en ce que $M^3$ est choisi parmi le zinc, le cérium, le zirconium, le cobalt, le nickel et le platine, et leurs mélanges.

7. Procédé selon la revendication 1, caractérisé en ce que $M^4$ est choisi parmi l'indium, l'argent, le magnésium, le calcium, le strontium, et le baryum, et leurs mélanges.

8. Procédé selon la revendication 7, caractérisé en ce que $M^4$ est choisi parmi l'argent, le calcium, le strontium et le baryum, et leurs mélanges.

9. Procédé selon la revendication 1, caractérisé en ce que la vapeur d'eau est introduite avec le toluène en quantité suffisante pour fournir un rapport molaire vapeur d'eau/toluène compris entre 0,1 et 10.

10. Procédé selon la revendication 1, caractérisé en ce que a et b dans la formule empirique sont 0,5 à 5.

11. Procédé selon la revendication 1, caractérisé en ce que la mise en contact entre le toluène et la composition minérale métal/oxygène est effectuée pendant une période comprise entre 1 seconde et 12 secondes.

12. Procédé selon la revendication 1, caractérisé en ce que la température est comprise entre 500°C et 600°C.

13. Procédé selon la revendication 1, caractérisé en ce que la réaction du déhydrocouplage est conduite dans un mode de fonctionnement stoechiométrique en l'absence d'oxygène libre ajouté.

14. Procédé selon la revendication 1, caractérisé en ce qu'un produit réagissant choisi dans le groupe se consistant d'oxygène et d'un gaz contenant de l'oxygène est introduit avec le toluène.

15. Procédé selon la revendication 14, caractérisé en ce que l'oxygène et un gaz contenant l'oxygène sont introduits en quantité suffisante pour conduire la réaction de déhydrocouplage dans un mode catalytique de fonctionnement.

16. Procédé selon la revendication 15, caractérisé en ce que l'oxygène et un gaz contenant l'oxygène sont introduits en quantité suffisante pour fournir un rapport molaire toluène/oxygène compris entre environ 1 et 8.

17. Procédé selon la revendication 14, caractérisé en ce que de l'oxygène et un gaz contenant de l'oxygène sont introduits en quantité suffisante pour conduire la réaction de déhydrocouplage dans un mode combiné catalytique/stoechiométrique de fonctionnement.

18. Procédé selon la revendication 1, caractérisé en ce que la composition minérale métal/oxygène est mélangée avec une matière de support.

19. Procédé selon la revendication 18, caractérisé en ce que la matière de support est un aluminate métallique.

20. Procédé selon la revendication 19, caractérisé en ce que l'aluminate métallique est l'aluminate de calcium.

21. Procédé selon la revendication 1, caractérisé en ce que la réaction de déhydrocouplage est conduite pour un niveau de conversion du toluène d'environ 20 à environ 55%.

22. Procédé selon l'une quelconque des revendications 1 à 16, caractérisé en ce que le produit déhydrocouplé du toluène est le stilbène.